# EUROPEAN PATENT SPECIFICATION

(11) **EP 4 243 814 B1**
(45) Date of publication and mention of the grant of the patent: **04.06.2025**
(21) Application number: 21892772.1
(22) Date of filing: 10.11.2021
(51) Int. Cl.: A61K 31/337, A61K 31/4155, A61K 31/713, A61K 9/00, A61P 1/16, A61P 9/00, A61P 25/00, A61P 25/28, A61P 35/00, A61K 31/4184, A61K 45/06

(54) **TREATMENT OF CONDITIONS WITH ABNORMAL HSP60/CLPP EXPRESSION AND/OR BINDING**
BEHANDLUNG VON ERKRANKUNGEN MIT ABNORMALER HSP60/CLPP-EXPRESSION UND/ODER -BINDUNG
TRAITEMENT D'ÉTATS AVEC UNE EXPRESSION ET/OU UNE LIAISON ANORMALE DE HSP60/CLPP

(30) Priority: 11.11.2020 US 202063112613 P
(43) Date of publication of application: 20.09.2023
(73) Proprietor: Health Research, Inc., Buffalo, NY 14263 (US)
(72) Inventor: CHANDRA, Dhyan, Buffalo, NY 14263 (US); KUMAR, Rahul, Buffalo, NY 14263 (US); YADAV, Neelu, Buffalo, NY 14263 (US)
(74) Representative: FRKelly
(86) International application number: PCT/US2021/058863
(87) International publication number: WO 2022/103897

(56) References cited:
- WO-A1-2019/112996
- US-A1- 2016 015 733
- US-A1- 2019 099 453
- US-A1- 2020 061 076
- DEEPA SATHYASEELAN S ET AL: "Down-regulation of the mitochondrial matrix peptidase ClpP in muscle cells causes mitochondrial dysfunction and decreases cell proliferation", FREE RADICAL BIOLOGY & MEDICINE, ELSEVIER INC, US, vol. 91, 23 December 2015 (2015-12-23), pages 281 - 292, XP029428370, ISSN: 0891-5849, DOI: 10.1016/J.FREERADBIOMED.2015.12.021
- KENNY TIMOTHY C ET AL: "From discovery of the CHOP axis and targeting ClpP to the identification of additional axes of the UPRmt driven by the estrogen receptor and SIRT3", JOURNAL OF BIOENERGETICS AND BIOMEMBRANES, PLENUM PUBLISHING, NEW YORK, NY, US, vol. 49, no. 4, 10 August 2017 (2017-08-10), pages 297 - 305, XP036307404, ISSN: 0145-479X, [retrieved on 20170810], DOI: 10.1007/S10863-017-9722-Z
- DATABASE PubChem Compound ANONYMOUS: "N-(1H-benzimidazol-2-ylmethyl)-1-[3-ethoxy-4-(thiophen-2-ylmethoxy)phenyl]methanamine | C22H23N3O2S - PubChem", XP055950369, retrieved from NCBI Database accession no. CID 9113063

## Description

### CROSS REFERENCE TO RELATED APPLICATION

This application claims priority to U.S. Provisional Patent Application No. 63/112,613, filed on November 11, 2020.

### STATEMENT REGARDING FEDERALLY SPONSORED RESEARCH

This invention was made with government support under grant no. CA160685 awarded by the National Institutes of Health. The government has certain rights in the invention.

### BACKGROUND OF THE DISCLOSURE

Prostate cancer (PCa) is the second most diagnosed cancer and third leading cause of cancer related death among American men. First-line treatment regimens for advanced PCa typically target and antagonize the androgen receptor (AR) signaling axis, the main driver of PCa growth. Although current therapies initially alleviate tumor burden, PCa typically recurs with a hormone-resistant phenotype that renders AR-targeting drugs ineffective. Hormone-refractive PCa is treated by taxanes-based chemotherapy, but this is largely palliative. Emergence of neuroendocrine PCa histological variants in some patients relapsing from hormone therapy pose another serious challenge because these cancers are no longer dependent on AR signaling.

Previous studies have demonstrated that mitochondria play important roles in the development of aggressive PCa, which may be attributed to their role in adapting to oxidative stress. Mitochondrial DNA (mtDNA) mutation-induced mitochondrial dysfunction has also been reported in aggressive PCa. To adapt to this pervasive mitochondrial stress, mammalian cells have developed a unique response system known as the mitochondrial unfolded protein response (UPR^{mt}). UPR^{mt} is a stress-induced response, which activates mitochondrial-specific chaperones and proteases to maintain mitochondrial homeostasis. UPR^{mt} is hyperactive and its components are upregulated in various cancers.

There are primarily two major mitochondria specific chaperones identified as heat shock proteins 60 and 10 (HSP60 and HSP10). More than 26 mitochondrial proteases have been identified in mammalian cells with LONP1 and caseinolytic protease proteolytic subunit (ClpP) playing prominent roles. HSP60 is encoded by HSPD1 gene, and is a mitochondrial chaperonin that properly folds nascent or denatured polypeptides. HSP60 monomers self-assemble within the mitochondrion to form a tetradecameric barrel. Damaged or nascent proteins bind to the apical domain of HSP60 within the core of the hydrophobic barrel. The addition of ATP and chaperonin HSP10 induces a charge turnover within the core of the barrel that leads to a conformational shift, which induces protein folding. HSP60 has recently been established as a protein-of-interest in various human diseases including cancer. Increased HSP60 expression predicts poor survival in advanced serous ovarian cancer; inhibits cell death in various tumor cells; is overexpressed in metastatic colon cancer; and is upregulated in gastric cancer cells where it inhibits apoptosis and induces migration. Increased expression of HSP60 correlates with aggressive phenotypes in PCa. These findings establish that HSP60 plays a critical role in maintaining mitochondrial function through the UPR^{mt}. Further, its up regulation in cancer suggests it may facilitate cancer cell growth and survival.

ClpP is a highly conserved mitochondrial serine protease and plays an important role in degradation of unfolded or misfolded proteins. ClpP exists as a heptamer in human mitochondria and relies on AAA+ chaperone ClpX plus ATP to be proteolytically active. ClpP-silencing sensitizes cervical carcinoma cells to the chemotherapeutic agent cisplatin by reducing platinum binding to mtDNA. ClpP is upregulated in primary acute myeloid leukemia (AML) specimens and loss of ClpP significantly decreases viability of AML cells. These findings suggest that both protein folding and protein degradation machinery may be critical in alleviating mitochondrial stress. However, it is not clear whether HSP60:HSP 10 folding machinery alone is sufficient or if it requires association with proteases such as ClpP in maintaining UPR^{mt} in cancer.

Kenny Timothy C et al. (JOURNAL OF BIOENERGETICS AND BIOMEMBRANES, 2017, vol. 49, no. 4, pages 297-305) disclose *inter alia* that knockdown of ClpP led to reduced tumor cell proliferation.

Based on the foregoing, there is an ongoing and unmet need for additional therapeutic targets beyond the AR signaling axis in order to improve patient outcomes, such as inhibitors of PCa cell growth that are effective on androgen nonresponsive prostate cancer cells.

### SUMMARY OF THE DISCLOSURE

It is to be understood that any reference to a method of treatment of a human or animal body, or disclosures thereof, are to be regarded as referring to a compound for such a use.

The present disclosure provides compositions for use in methods for treating an individual in need of treatment with a compound that inhibits the growth of cancer cells. The methods may be used to disrupt mitochondrial unfolded protein response. A compound of this disclosure binds to heat shock protein 60 (HSP60) and inhibits the interaction of HSP60 to ClpP, thus interrupting the mitochondrial unfolded protein response. Without intended to be bound by any particular theory, it is considered targeting HSP60-ClpP mediated UPR^{mt} may provide a novel therapeutic approach for treating advanced prostate cancers that are no longer sensitive to AR targeted therapies.

In an aspect, the present disclosure provides compositions for use in a method for treating an individual in need of treatment having a condition in which HSP60/ClpP expression and/or binding is abnormal, the use comprising administering to the individual in need of treatment a therapeutically effective amount of the composition comprising a compound having the following structure:

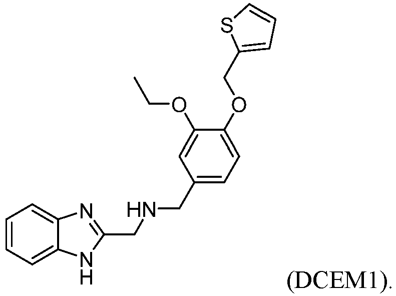

Without intending to be bound by any particular theory, it is considered DCEM1 binds to the apical domain of HSP60, a region that is critical for interacting with ClpP, and disrupts mitochondrial protein homeostasis leading to cancer cell death.

In an aspect, the present disclosure discloses methods of using compositions comprising DCEM1. A method may use a composition of the present disclosure. The compositions of the present disclosure are used to treat cancers (e.g., prostate cancer, leukemia, lung cancer, dermatological cancer, premalignant lesions of the upper digestive tract, malignancies of the brain, malignancies of the breast, and the like, and combinations thereof). For example, one or more compositions of the present disclosure can be used to treat cancer and/or induce selective inhibition of the UPR^{mt}. A method of this disclosure may be carried out in combination with one or more known therapy(ies), including, but not limited to, surgery, radiation therapy, chemotherapy, photodynamic therapy, and/or immunotherapy.

The scope of the invention is set out in the accompanying claims.

### BRIEF DESCRIPTION OF THE FIGURES

For a fuller understanding of the nature and objects of the disclosure, reference should be made to the following detailed description taken in conjunction with the accompanying figures.

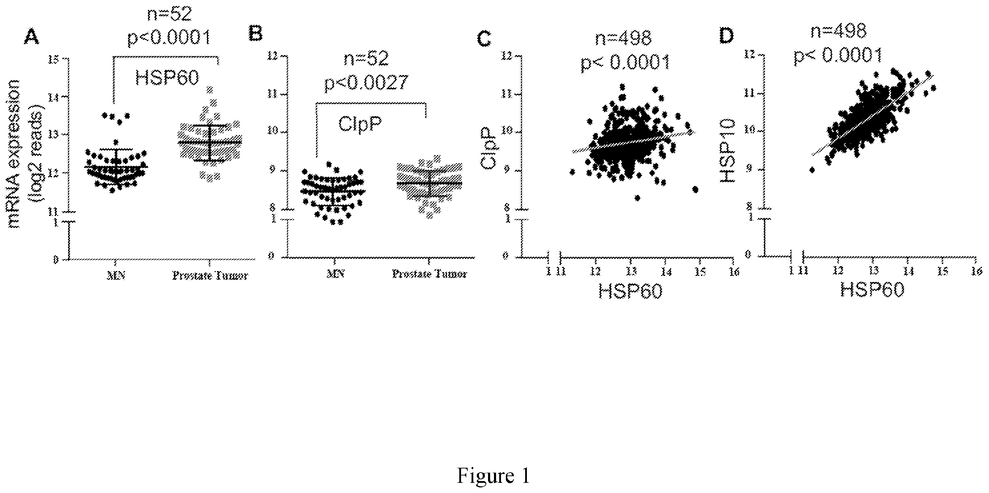

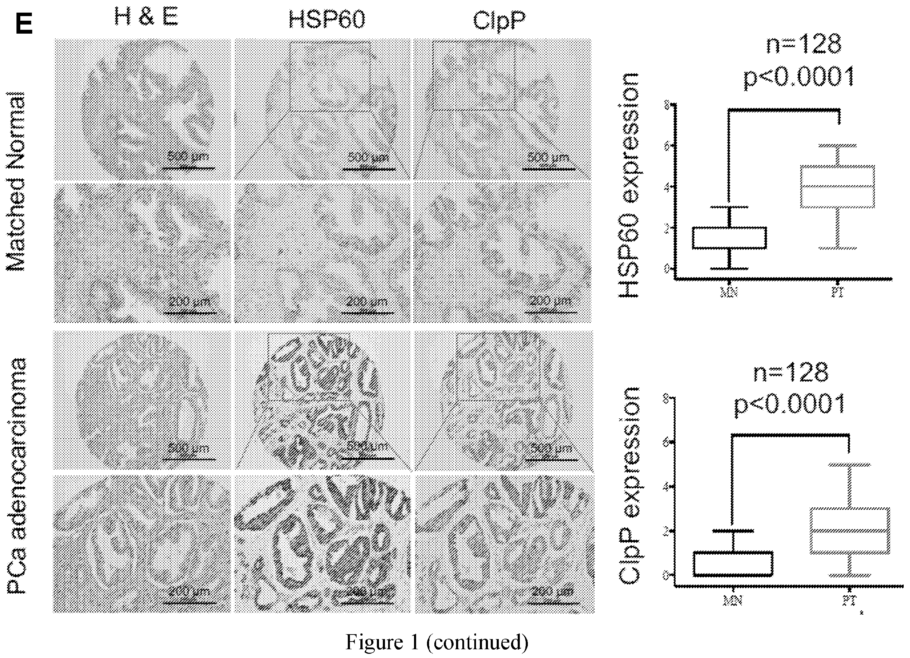

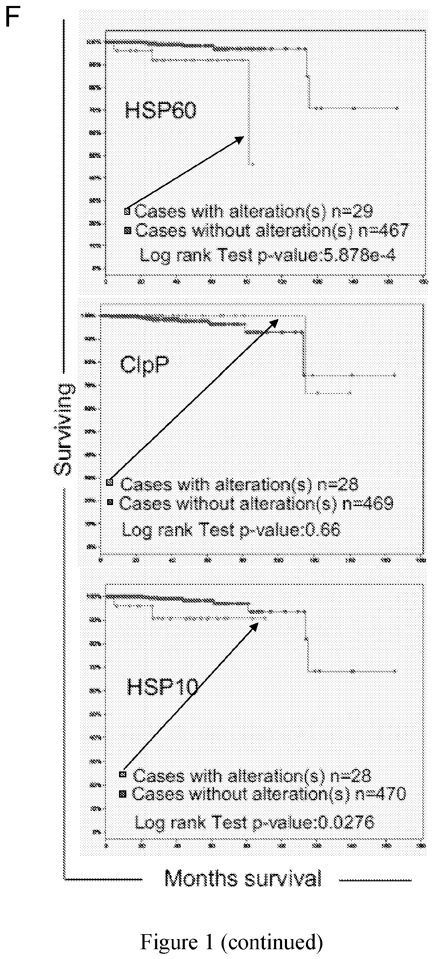

Figure 1. UPR^{mt} components are upregulated in PCa and associate with poor prognosis in PCa Patients. (A) TCGA Analysis of HSP60 transcript reads in prostate tumors compared to matched normal counterparts. (B) TCGA Analysis of ClpP transcript reads in prostate tumors compared to matched normal counterparts. (C) Correlative analysis between HSP60 and ClpP transcript reads from TCGA. (D) Correlative analysis between HSP60 and HSP10 transcript reads from TCGA. (E) Representative images from PCa TMA that were staine with H&E, HSP60, or ClpP. Images were scored and quantified. (F) Kaplan Meier curves depicting overall survival in patients with altered expression of HSP60, ClpP or HSP10 compared to patients with no alterations in these genes. Data are mean +/- SD (n = at least 3). * p<0.05.

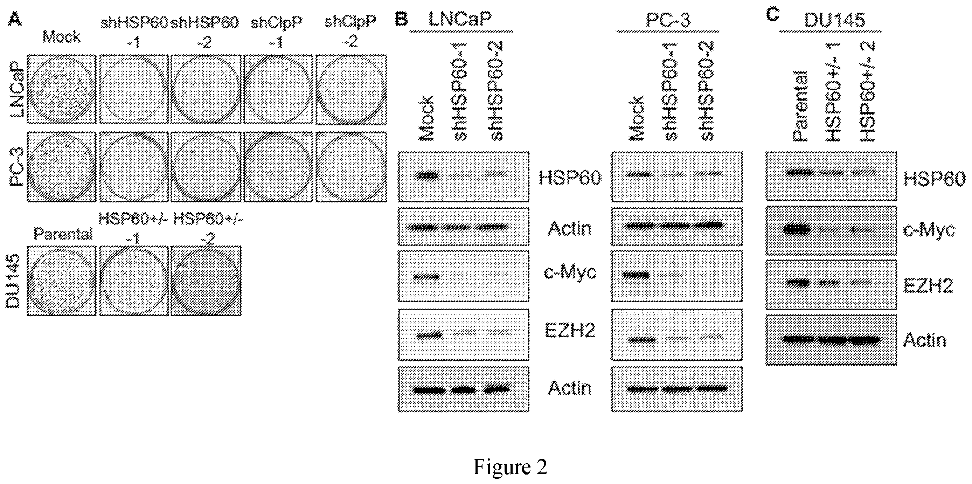

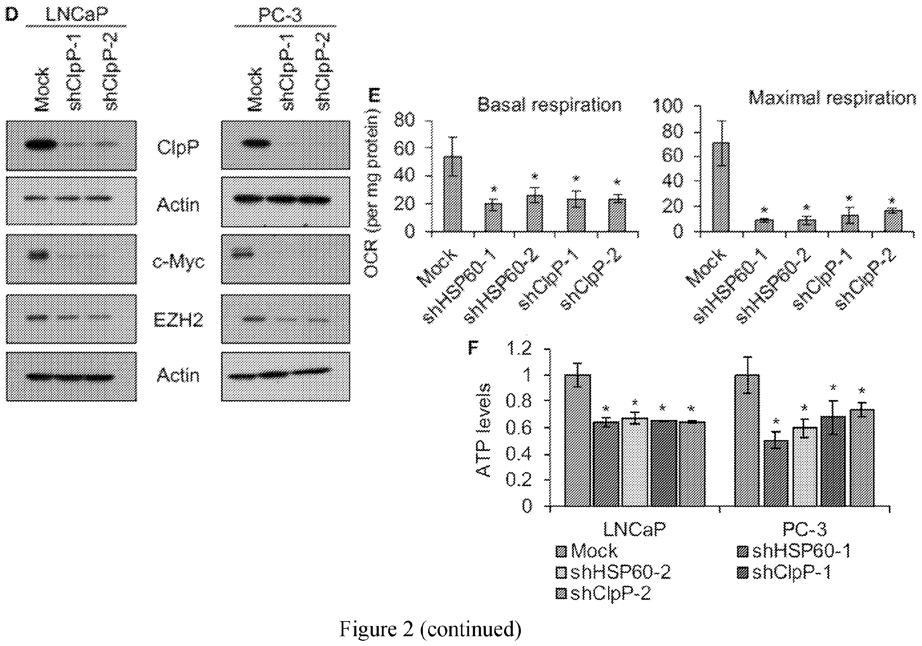

Figure 2. HSP60 and ClpP silencing inhibit c-Myc, EZH2 signaling and induce metabolic stress in PCa cells. (A) Clonogenic assays were performed in HSP60 and ClpP silenced LNCaP and PC-3 cells and in DU145 HSP60± cells as described herein. (B) Protein expression level of c-Myc and EZH2 in HSP60 silenced LNCaP and PC-3 cells. (C) Protein expression level of c-Myc and EZH2 in DU145 HSP60± cells. (D) Protein expression level of c-Myc and EZH2 in ClpP silenced LNCaP and PC-3 cells. (E) Oxygen consumption rate (OCR) was analyzed in HSP60 and ClpP silenced PC-3 cells by seahorse analyzer and represented as basal and maximal respiration rate. (F) ATP levels were estimated in HSP60 and ClpP silenced LNCaP and PC-3 cells as described in material and methods. In (F), the bars from left to right in each series are Mock, shHSP60-2, shClpP-2, shHSP60-1, and shClpP-1.

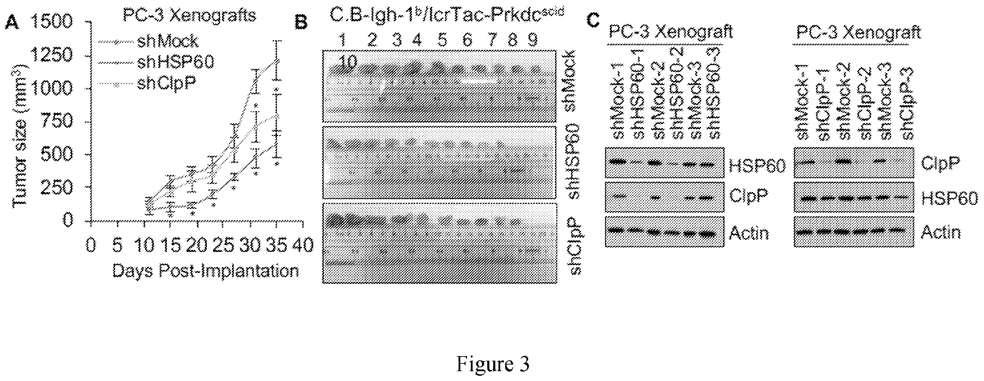

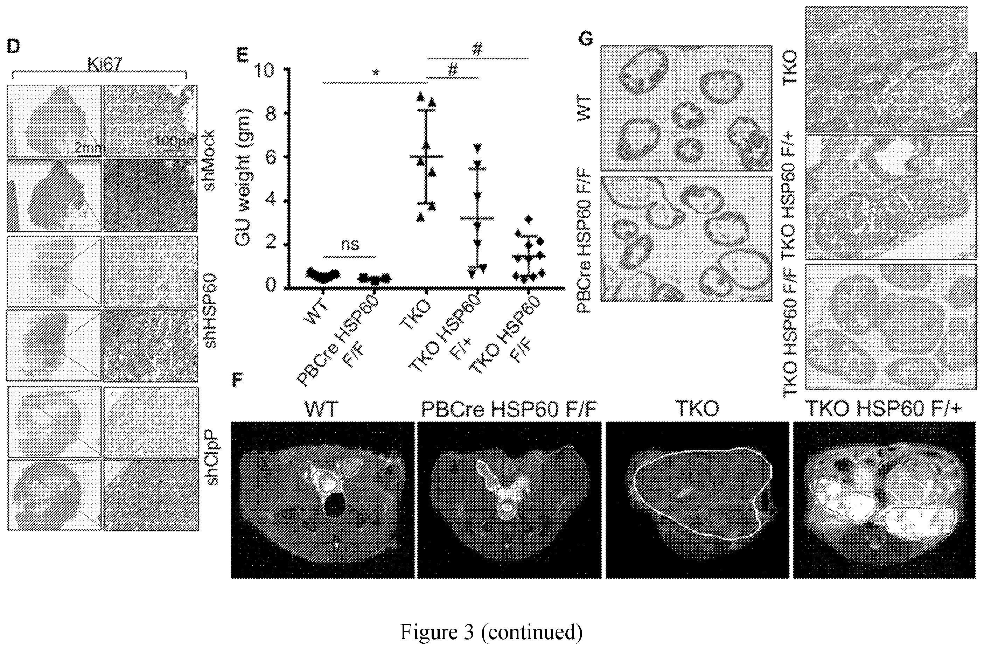

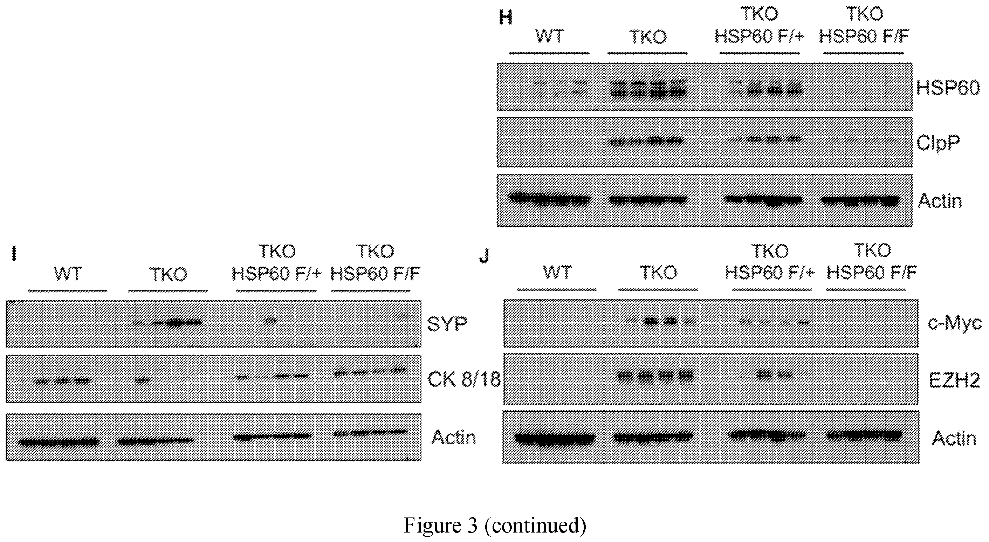

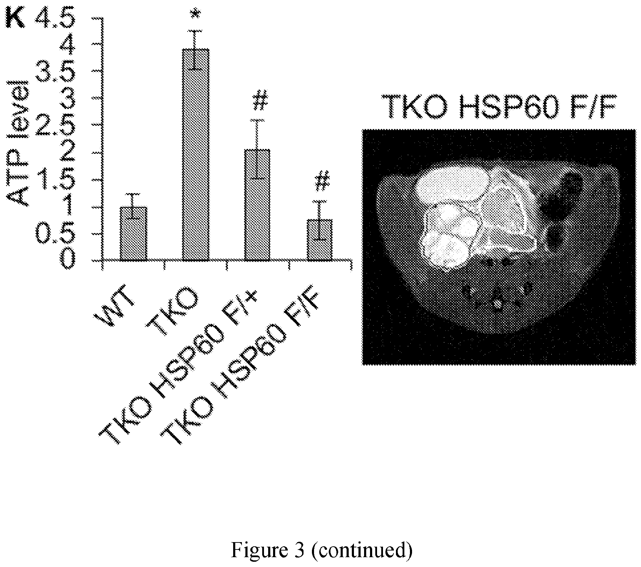

Figure 3. Silencing of HSP60 and ClpP reduces tumor burden *in vivo.* (A-C) PC3 cells with either HSP60 or ClpP knocked down were transplanted into SCID mice. Tumor size was checked every 4 days. Xenografts were harvested after 35 days and photographed (B) and HSP60 and ClpP knockdown efficiency was determined via western blot (C). Data are mean +/-SD. * p<0.05. (D) HSP60 and ClpP knockdown PC3 xenografts were sectiond and subjected to Ki-67 IHC and H&E staining. (E) WT; PBCre4: HSP60 F/F; TKO, TKO HSP60 F/+ and TKO HSP60 F/F prostate were imaged by MRI as described in material and methods and represented as prostate tumor volume. (F) WT; PBCre4: HSP60 F/F; TKO, TKO HSP60 F/+ and TKO HSP60 F/F prostate tissue and tumors were harvested at 16 weeks of age and whole genitourinary tract was weighed. (G) WT; PBCre4: HSP60 F/F; TKO, TKO HSP60 F/+ and TKO HSP60 F/F prostate tissue and tumors were harvested at 16 weeks of age and representative H & E images have been shown. (H-J) WT; PBCre4: HSP60 F/F; TKO, TKO HSP60 F/+ and TKO HSP60 F/F prostate tissue and tumors were harvested at 16 weeks of age and whole cell lysate were prepared and analyzed for- HSP60 and ClpP expression (H); SYP (neuroendocrine marker) and CK 8/18 (epithelial marker) expression (I) and c-Myc and EZH2 expression (J) by immunoblotting. (K) ATP levels were analyzed in WT; PBCre4: HSP60 F/F; TKO, TKO HSP60 F/+ and TKO HSP60 F/F prostate tissue. Data are mean +/- SD (n = at least 3). * p<0.05 and # p<0.05 compared to indicated groups/controls.

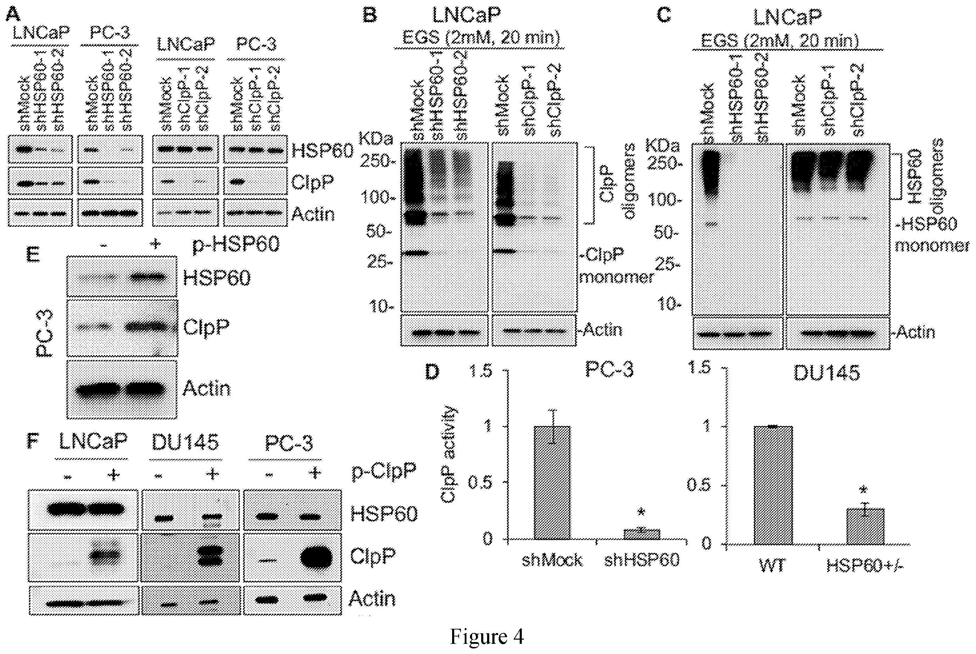

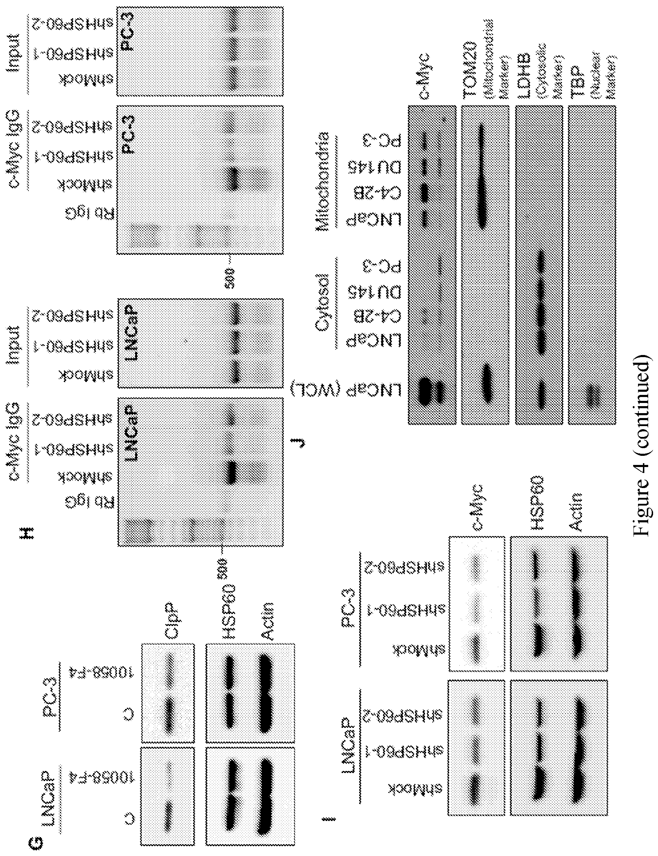

Figure 4. HSP60 regulate ClpP expression but not vice versa. (A) HSP60 and ClpP stable knockdown LNCaP and PC-3 cells were generated using shRNA and analyzed for HSP60 and ClpP expression levels. Actin serves as loading control. (B-C) HSP60 and ClpP knockdown LNCaP and PC-3 cells were crosslinked with EGS, lysed and protein samples run on an SDS-PAGE gel as described in material and methods. Membranes were probed with either ClpP or HSP60 antibody to analyze the oligomerization status of these proteins. (D) Enzymatic activity of ClpP was assayed from mitochondrial pellets isolated from HSP60 knockdown PC-3 cells and DU145 Hsp60^{+/-}cells. (E) HSP60 was overexpressed in PC-3 cells and analyzed for ClpP expression. Actin serves as loading control. (F) ClpP was overexpressed in LNCaP, DU145 and PC-3 cells and analyzed for HSP60 expression. Actin serves as loading control. (G) LNCaP and PC-3 cells were treated with c-Myc inhibitor (10058-F4, 50 µM) for 24 hrs. Whole cell lysates were prepared and analyzed for HSP60 and ClpP expression. Actin serves as loading control. (H) c-Myc binding efficiency with ClpP promoter in HSP60 knockdown LNCaP and PC-3 cells was determined using chromatin immunoprecipitation (ChIP) assay using specific primers. (I) c-Myc expression in HSP60 and ClpP stable knockdown LNCaP and PC-3 cells. Actin serves as loading control. (J) c-Myc expression in cytosolic and mitochondrial extracts from PCa cells (LNCaP, C4-2B, DU145 and PC-3). TOM20, LDHB and TBP used as markers for mitochondrial, cytosolic and nuclear to determine purity of the extracts. Data are mean +/-SD (n = at least 3). * p<0.05.

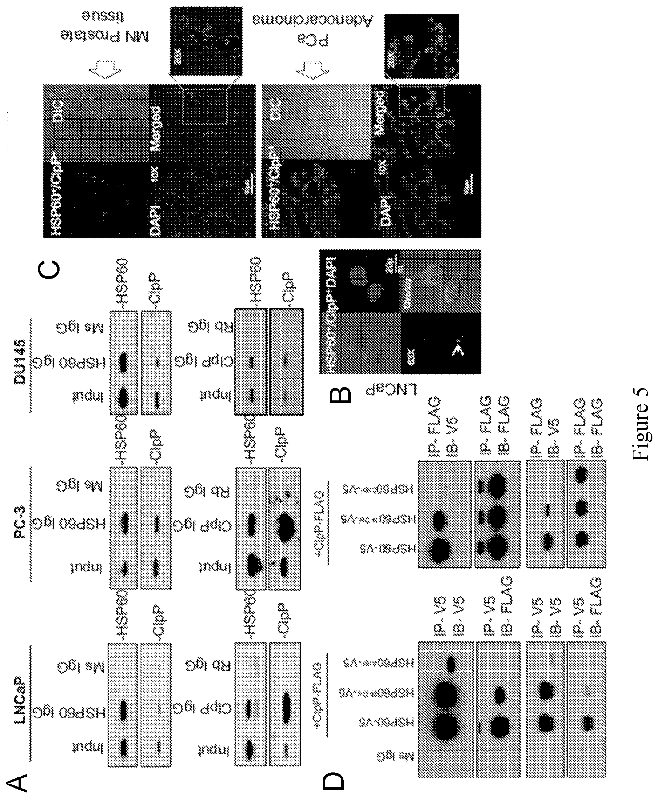

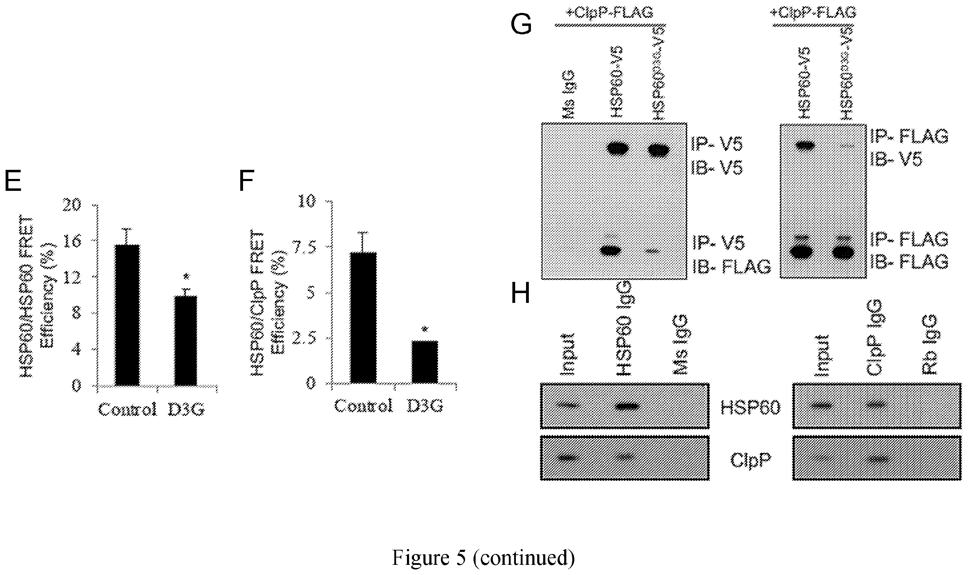

Figure 5. Human Hsp60 and ClpP directly interact within the mitochondrial matrix. (A) Co-IPs performed between HSP60 and ClpP in LNCaP, PC-3 and DU145 cells. (B) Proximity Ligation Assay (PLA) between HSP60 and ClpP in LNCaP cells. (C) Proximity Ligation Assay (PLA) between HSP60 and ClpP performed in TMA (n=128) constructed from normal prostate (MN) and prostate adenocarcinoma tissue. (D) Mitochondrial localization signal (HSP60N-Del) and apical domain (HSP60Δapi) were deleted from HSP60 construct with V5 tag and co-transfected with ClpP construct with FLAG tag in PC-3 cells. Co-IPs were performed using either V5 antibody or FLAG antibody. (E) FRET analysis was performed to analyze the effect of D3G mutation on HSP60/HSP60 interaction as described in material and methods. (F) FRET analysis was performed to analyze the effect of D3G mutation on HSP60/ClpP interaction as described in material and methods. (G) D3G mutant form of HSP60 construct with V5 tag was co-transfected with ClpP construct with FLAG tag in PC-3 cells. Co-IPs were performed using either V5 antibody or FLAG antibody. (H) Co-IPs performed between HSP60 and ClpP in TKO prostatic tumor tissue. Data are mean +/- SD (n = at least 3). * p<0.05.

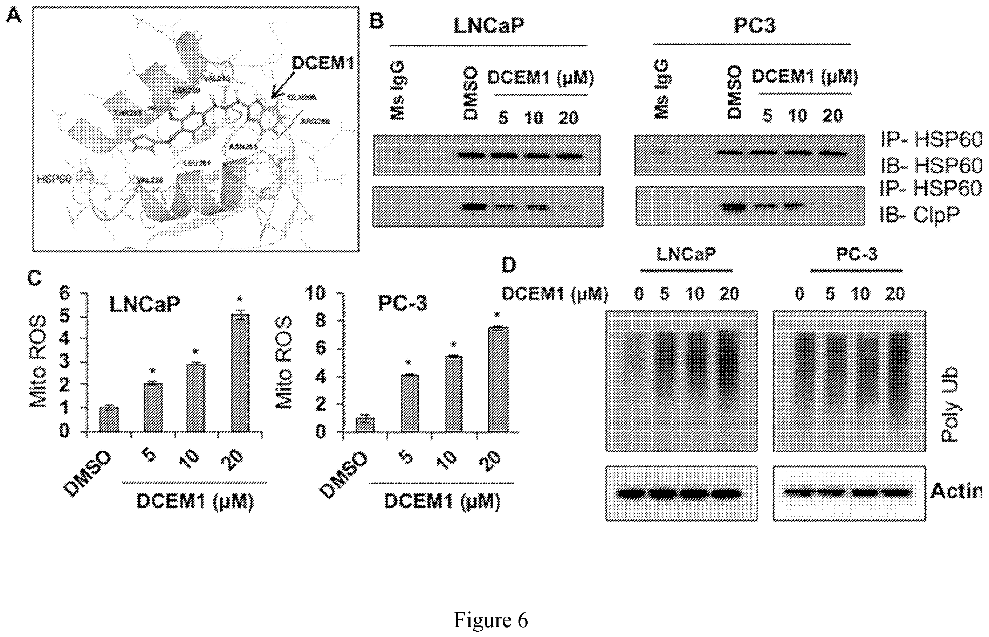

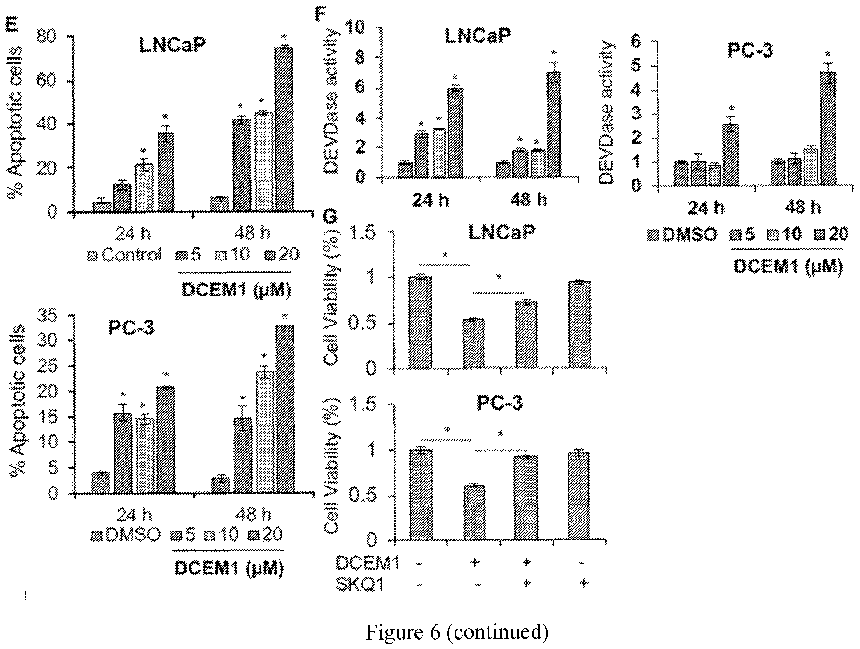

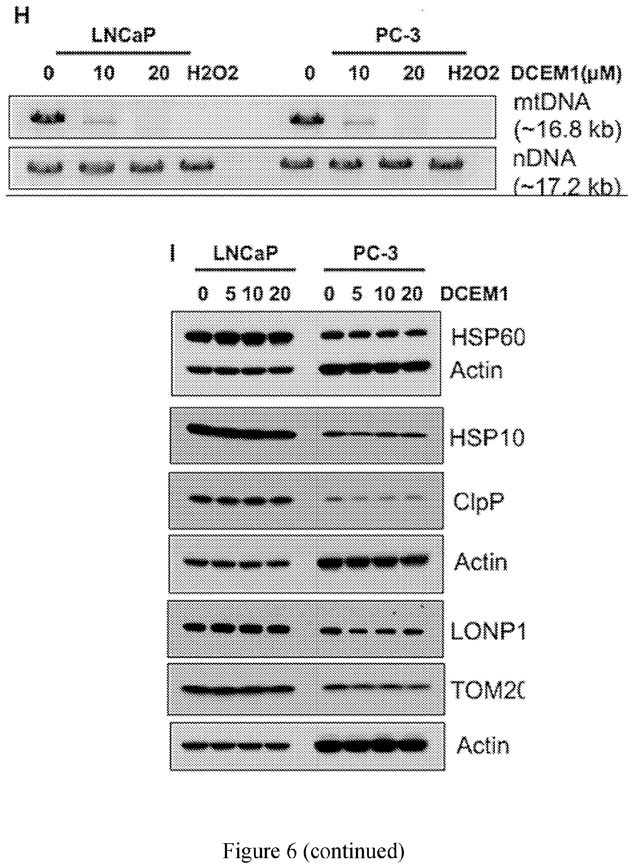

Figure 6. Pharmacological inhibition of HSP60-ClpP interaction induces UPR^{mt} and PCa cell death. (A) Docking of DCEM1 into apical domain of HSP60. (B) LNCaP and PC-3 cells were treated with DCEM1 for 24 hours and HSP60-ClpP interaction was analyzed by co-immunoprecipitation assay. (C) LNCaP and PC-3 cells were treated with DCEM1 for 24 hours and mitoROS levels were analyzed by flow cytometry using mitoSOX dye. (D) LNCaP and PC-3 cells were treated with DCEM1 for 24 hours and poly-Ub protein levels were analyzed by western blotting. (E) LNCaP and PC-3 cells were treated with DCEM1 for 24 and 48 hours and apoptotic cell populations were analyzed by flow cytometry using Annexin V FITC/PI method as explained in materials and methods. In (E) top panel, the bars in each series from left to right are control, 5, 10, and 20, and in (E) bottom panel, the bars in each series from left to right are DMSO, 5, 10, and 20. (F) LNCaP and PC-3 cells were treated with DCEM1 for 24 and 48 hours and lysates were prepared and analyzed for DEVDase activity using specific peptide substrate. In (F), the bars in each series from left to right are DMSO, 5, 10, and 20. (G) LNCaP and PC-3 cells were pre-treated with SKQ1 followed by DCEM1 treatment and analyzed for cell viability by MTT assay. (H) LNCaP and PC-3 cells were treated with either DCEM1 or H₂O₂ (200 µM) for 24 hours and total DNA were isolated and analyzed for mtDNA damage as described in materials and methods. (I) DCEM1 treatement failed to alter the expression of UPR^{mt} components including HSP60, HSP10, ClpP and LONP1.

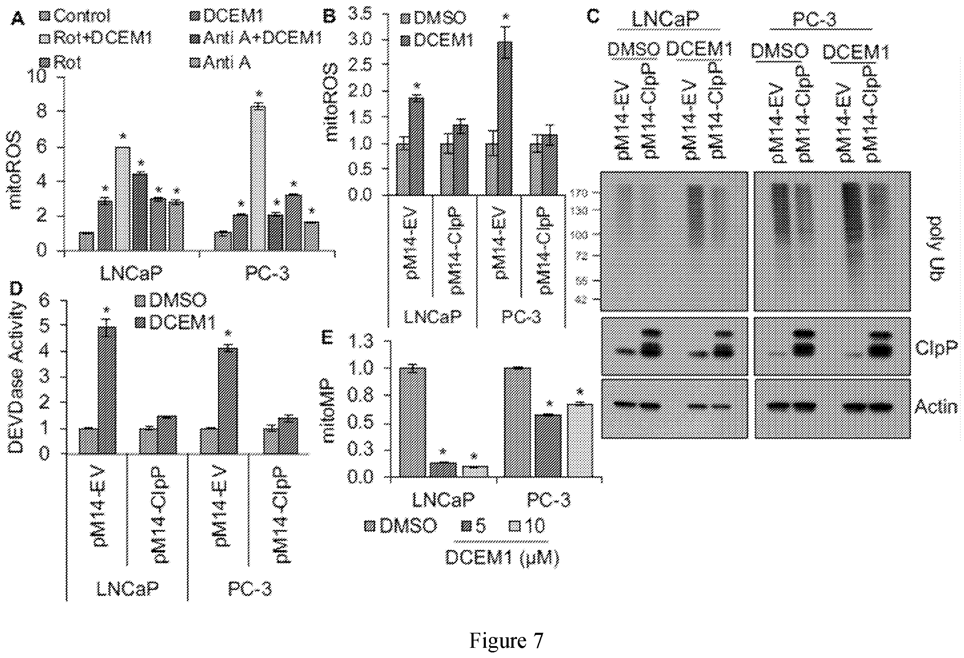

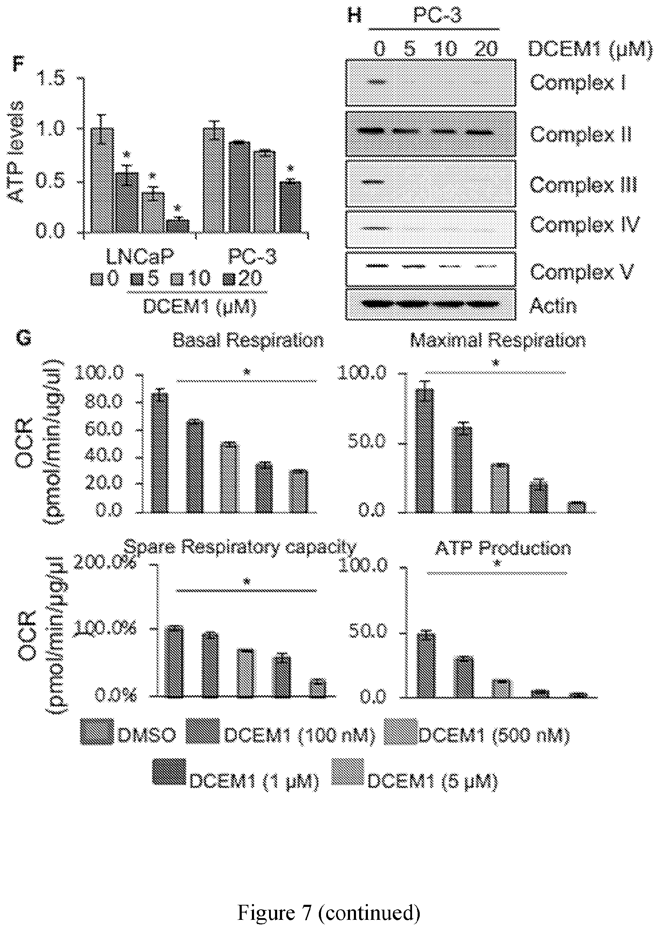

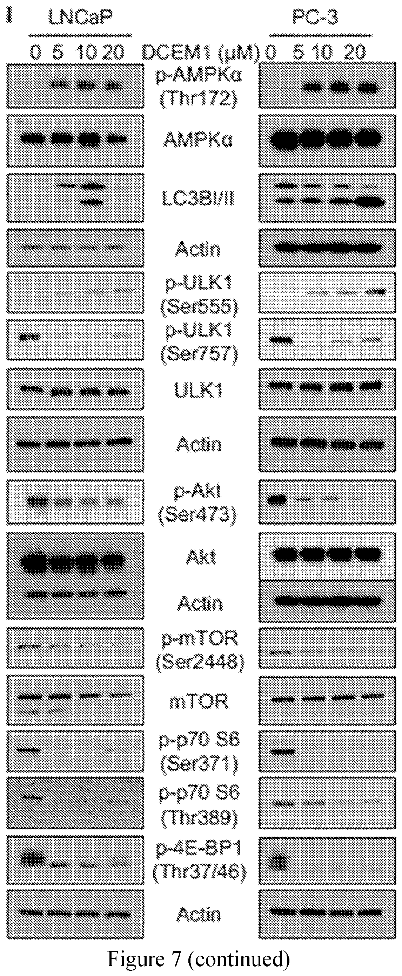

Figure 7. Pharmacological inhibition of HSP60-ClpP interaction induces metabolic stress in PCa cells. (A) LNCaP and PC-3 cells were pre-treated with either rotenone or antimycin-A followed by DCEM1 treatment and mitochondrial ROS was analyzed as described in material and methods (data represented as fold change compared to control). In (A), the bars in each series from left to right are control, DCEM1, Rot+DCEM1, Anti A+DCEM1, Rot, and Anti A (B) ClpP protein was over-expressed in LNCaP and PC-3 cells followed by DCEM1 treatment and mitochondrial ROS was analyzed as described in material and methods (Data represented as fold change compared to control). In (B), the bars in each series from left to right are DMSO and DCEM1. (C) ClpP protein was over-expressed in LNCaP and PC-3 cells followed by DCEM1 treatment and level of poly-ubiquitinated protein was analyzed by western blotting. (D) ClpP protein was over-expressed in LNCaP and PC-3 cells followed by DCEM1 treatment and DEVDase activity was analyzed as described in material and methods (Data represented as fold change compared to control). In (D), the bars in each series from left to right are DMSO and DCEM1. (E) Mitochondrial membrane potential (mitoMP) was analyzed in LNCaP and PC-3 cells treated with DCEM1 as described in material and methods (Data represented as fold change compared to control). In (E), the bars in each series from left to right are DMSO, 5, and 10. (F) ATP level was analyzed in LNCaP and PC-3 cells treated with DCEM1 as described in material and methods (Data represented as fold change compared to control). In (F), the bars in each series from left to right are 0, 5, 10, and 20. (G) Oxygen consumption rate was analyzed in PC-3 cells treated with DCEM1 as described in material and methods and represented as basal and maximal respiration rate; Spare respiratory capacity and ATP production potential. (In (G), the bars in each series from left to right are DMSO, DCEM1 (100 nM), DCEM1 (500 nM), DCEM1 (1 µM), and DCEM1 (5 µM). (H) Protein expression levels of nuclear encoded OXPHOS subunits were analyzed in PC-3 cells treated with DCEM1. Actin served as loading control. (I) AMPK and mTOR signaling pathways were analyzed in LNCaP and PC-3 cells treated with DCEM1. Actin served as loading control. Data are mean +/-SD (n = at least 3). * p<0.05.

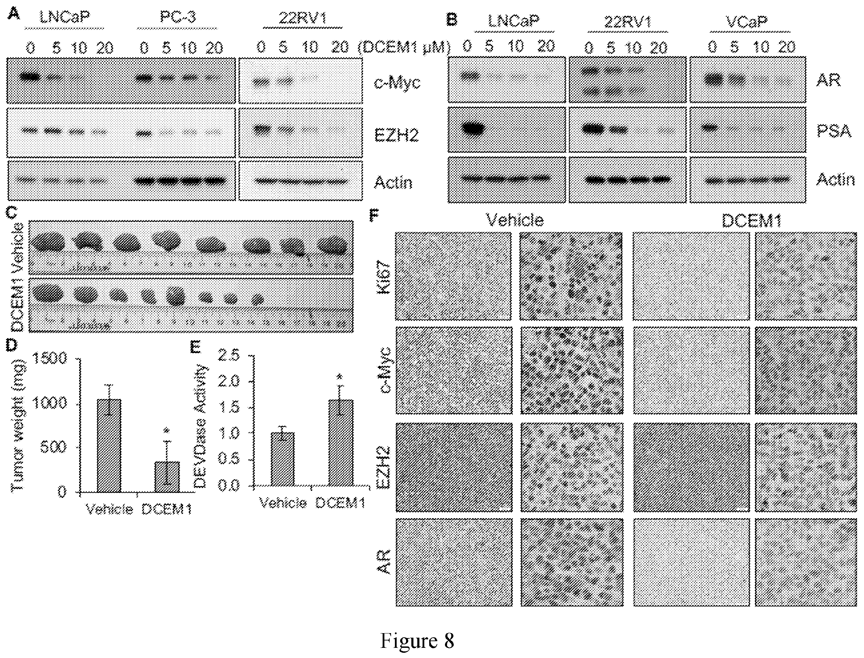

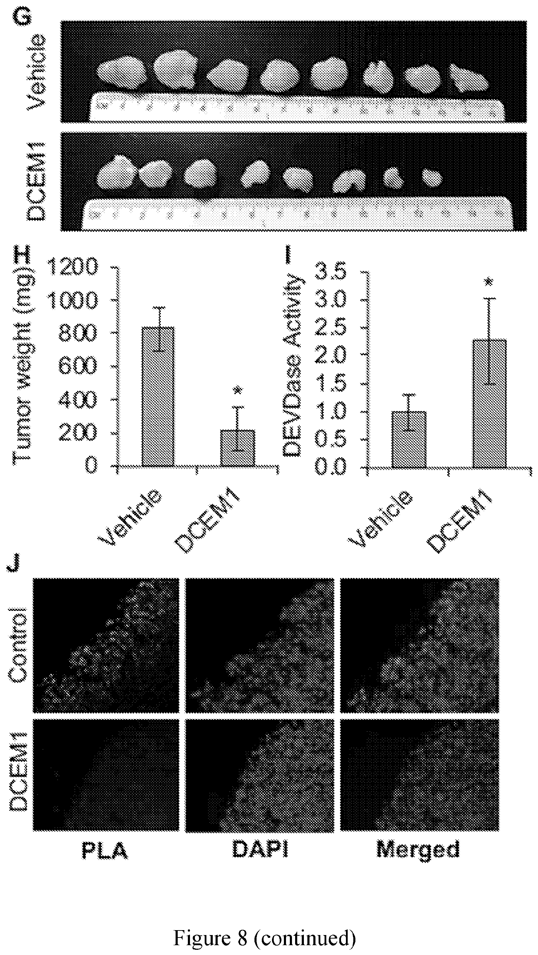

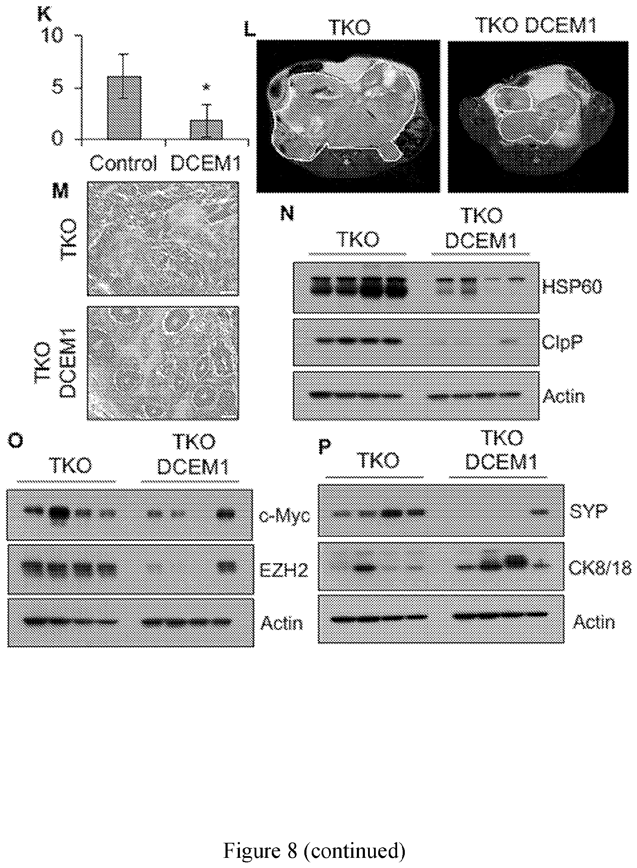

Figure 8. Pharmacological inhibition of HSP60-ClpP inhibit oncogenic signaling and PCa tumor growth *in vivo.* (A) LNCaP, PC-3 and 22RV1 cells were treated with DCEM1 and analyzed for c-Myc and EZH2 protein expression after 24 hours of treatment. (B) LNCaP, 22RV1 and VCaP cells were treated with DCEM1 and analyzed for AR and PSA protein expression after 24 hours of treatment. (C-D) 22RV1 cells (1 x 10⁶ cells) were mixed with matrigel (50:50 v/v) and injected into each flank of SCID mice and treated with DCEM1 (60 mg/kg bw) through IP route twice weekly and tumors were harvested, photographed (C) and weighed (D) at 30 days. (E) DEVDase activity were analyzed in 22RV1 xenograft tumor tissue following DCEM1 treatment and represented as fold change compared to control. (F) 22RV1 xenograft tissue were sectioned and expression of ki67, c-Myc and EZH2 proteins were analyzed by Immunohistochemistry as described in material and methods. (G-H) PC-3 cells (1 x 10⁶ cells) were mixed with matrigel (50:50 v/v) and injected into each flank of SCID mice and treated with DCEM1 (60 mg/kg bw) through IP route twice weekly and tumors were harvested, photographed (G) and weighed (H) at 35 days. (I) DEVDase activity were analyzed in PC-3 xenograft tumor tissue following DCEM1 treatment and represented as fold change compared to control. (J) PC-3 xenograft tumor tissue were fixed and sections were used for in situ PLA to analyze HSP60-ClpP interaction in these tissue samples. (K) PBCre4:PTEN F/F: Rb F/F: p53 F/F (TKO) animals were treated with either vehicle (Normal saline: DMSO: Kolliphor HS15; 70:5:200 v/v) or DCEM1 (60 mg/kg bw in vehicle) twice weekly from 10 weeks of age. All animals were sacrificed at 16 weeks of age and whole genitourinary tract was harvested and weighed. (L) PBCre4:PTEN F/F: Rb F/F: p53 F/F (TKO) animals were treated with either vehicle (Normal saline: DMSO: Kolliphor HS15; 70:5:200 v/v) or DCEM1 (60 mg/kg bw in vehicle) twice weekly from 10 weeks of age. All animals were sacrificed at 16 weeks of age and imaged by MRI. (M) PBCre4:PTEN F/F: Rb F/F: p53 F/F (TKO) animals were treated with either vehicle (Normal saline: DMSO: Kolliphor HS15; 70:5:200 v/v) or DCEM1 (60 mg/kg bw in vehicle) twice weekly from 10 weeks of age. All animals were sacrificed at 16 weeks of age and prostate tissue and tumors were harvested and representative H & E images have been shown. (N-P) PBCre4:PTEN F/F: Rb F/F: p53 F/F (TKO) animals were treated with either vehicle (Normal saline: DMSO: Kolliphor HS15; 70:5:200 v/v) or DCEM1 (60 mg/kg bw in vehicle) twice weekly from 10 weeks of age. All animals were sacrificed at 16 weeks of age and prostate tissue and tumors were harvested, whole cell lysate were prepared and analyzed for HSP60 and ClpP expression (N); c-Myc and EZH2 expression (O) and SYP and CK 8/18 expression (P) by immunoblotting. Data are mean +/- SD (n = at least 3). * p<0.05.

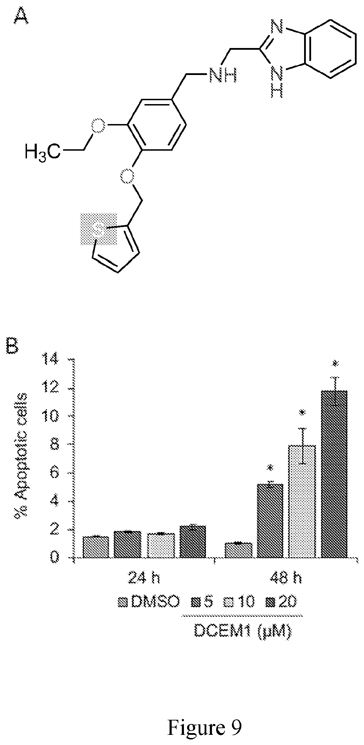

Figure 9. Structure of DCEM1 is shown in (A). (B) Human prostate epithelial non carcinoma RWPE-1 cells were treated with DCEM1 at indicated doses for 24 h and 48 h and apoptotic cells were quantified by Annexin V/PI labeling as described in material and methods. In (B), the bars in each series from left to right are DMSO, 5, 10, and 20.

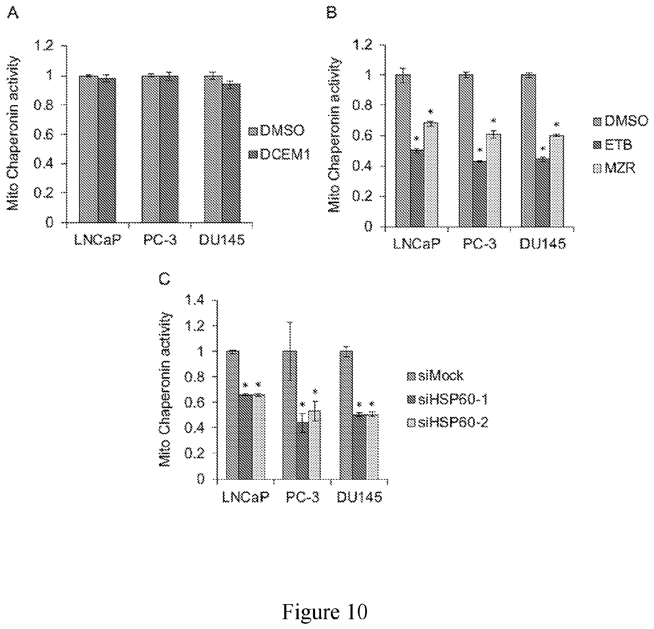

Figure 10. Mitochondrial Chaperonin Activity Assay (MiCAA). PCa cells were treated with either DCEM1 (A) or ETB or Mizoribine (B) for 24 h and mitochondrial (Mito) chaperonin activity was analyzed as described in material and methods. In another experiment, PCa cells were transfected with siRNA to target HSP60 mRNA and mitochondrial chaperonin activity was analyzed (C). In (A), the bars in each series from left to right are DMSO and DCEM1. In (B), the bars in each series from left to right are DMSO, ETB, and MZR. In (C), the bars in each series from left to right are siMock, siHSP60-1, and siHSP60-2.

### DETAILED DESCRIPTION OF THE DISCLOSURE

The technical disclosure set out below may, in some respects, go beyond the scope of the claims. Elements of the disclosure that do not fall within the scope of the claims are provided for information.

Although claimed subject matter will be described in terms of certain examples, other examples, including examples that do not provide all of the benefits and features set forth herein, are also within the scope of this disclosure. Various structural, logical, and process step changes may be made without departing from the scope of the disclosure.

Every numerical range given throughout this specification includes its upper and lower values, as well as every narrower numerical range that falls within it, as if such narrower numerical ranges were all expressly written herein. All ranges provided herein include all values that fall within the ranges to the hundredth decimal place, unless indicated otherwise.

The present disclosure provides methods and compositions for treating an individual in need of treatment with a compound that inhibits the growth of cancer cells. The methods may be used to disrupt mitochondrial unfolded protein response. In various examples, a compound of this disclosure binds to heat shock protein 60 (HSP60) and inhibits the interaction of HSP60 to ClpP, thus interrupting the mitochondrial unfolded protein response. Without intended to be bound by any particular theory, it is considered targeting HSP60-ClpP mediated UPR^{mt} may provide a novel therapeutic approach for treating advanced prostate cancers that are no longer sensitive to AR-targeted therapies.

Throughout this application, the singular form encompasses the plural and vice versa. All sections of this application, including any supplementary sections or figures, are fully a part of this application.

The term "treatment" as used herein refers to alleviation of one or more symptoms or features associated with the presence of the particular condition or suspected condition being treated. Treatment does not necessarily mean complete cure or remission, nor does it preclude recurrence or relapses. Treatment can be effected over a short term, over a medium term, or can be a long-term treatment, such as, within the context of a maintenance therapy. Treatment can be continuous or intermittent.

The term "therapeutically effective amount" as used herein refers to an amount of an agent sufficient to achieve, in a single or multiple doses, the intended purpose of treatment. The exact amount desired or required will vary depending on the particular compound or composition used, its mode of administration, patient specifics and the like. Appropriate effective amount can be determined by one of ordinary skill in the art informed by the instant disclosure using only routine experimentation.

In an aspect, the present disclosure provides compositions comprising a compound having the following structure: 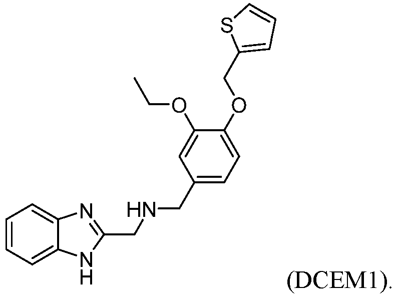 Without intending to be bound by any particular theory, it is considered DCEM1 binds to the apical domain of HSP60, a region that is critical for interacting with ClpP, and disrupts mitochondrial protein homeostasis leading to cancer cell death.

Compositions may comprise DCEM1 and one or more pharmaceutically acceptable carriers. Non-limiting examples of carriers include solutions, suspensions, emulsions, solid injectable compositions that are dissolved or suspended in a solvent before use, and the like. The compositions may be prepared by dissolving, suspending, or emulsifying one or more of the active ingredients in a diluent. Examples of diluents, include, but are not limited to distilled water, physiological saline, vegetable oil, alcohol, dimethyl sulfoxide, and combinations thereof. Further, the compositions may contain stabilizers, solubilizers, suspending agents, emulsifiers, soothing agents, buffers, preservatives, etc. The compositions may be sterilized in the final formulation step or prepared by sterile procedure.

The composition of the disclosure may also be formulated into a sterile solid preparation, for example, by freeze-drying, and can be used after sterilized or dissolved in sterile injectable water or other sterile diluent(s) immediately before use. Additional examples of pharmaceutically acceptable carriers include, but are not limited to, sugars, such as lactose, glucose, and sucrose; starches, such as corn starch and potato starch; cellulose, including sodium carboxymethyl cellulose, ethyl cellulose, and cellulose acetate; powdered tragacanth; malt; gelatin; talc; excipients, such as cocoa butter and suppository waxes; oils, such as peanut oil, cottonseed oil, safflower oil, sesame oil, olive oil, corn oil, and soybean oil; glycols, such as propylene glycol; polyols, such as glycerin, sorbitol, mannitol, and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations; and combinations thereof. Additional non-limiting examples of pharmaceutically acceptable carriers can be found in: Remington: The Science and Practice of Pharmacy (2012) 22nd Edition, Philadelphia, PA. Lippincott Williams & Wilkins.

In an aspect, the present disclosure provides methods of using compositions comprising DCEM1. A method may use a composition of the present disclosure.

The compositions of the present disclosure are suitable in methods to treat cancers (e.g., prostate cancer, leukemia, lung cancer, dermatological cancer, premalignant lesions of the upper digestive tract, malignancies of the brain, malignancies of the breast, and the like, and combinations thereof). For example, one or more compositions of the present disclosure can be used to treat cancer and/or induce selective inhibition of the UPR^{mt} and/or inhibit the growth/replication of malignant cells and/or metastasis of cancers. A method of this disclosure may be carried out in combination with one or more known therapy(ies), including, but not limited to, surgery, radiation therapy, chemotherapy, photodynamic therapy, and/or immunotherapy.

In various examples, the composition of the present disclosure may be administered in combination with one or more chemotherapy drugs. The composition may be administered sequentially or concurrently with one or more chemotherapy drugs. The sequential administration of the composition and one or more chemotherapy drugs may be separated by seconds, minutes, hours, days, or weeks. Examples of chemotherapy drugs that may be used in combination with the composition include, but are not limited to docetaxel, paclitaxel, enzalutamide, cabazitaxel, abiraterone, etoposide, doxorubicin, tyrosine kinase inhibitors, and the like, and combinations thereof. For example, using a composition comprising DCEM1 in combination with one or more chemotherapy drugs may increase the efficacy of the one or more chemotherapy drugs.

The methods of the present disclosure may be used to inhibit HSP60 from binding ClpP, thus disrupting the UPR^{mt} activity. The UPR^{mt} activity may be altered *in vitro* or *in vivo.* Methods of the present disclosure may comprise administering a composition of the present disclosure to an individual exhibiting abnormal HSP60/ClpP expression and/or binding. Examples of conditions in which the present methods and compositions can be used includes, but is not limited to, any condition in which HSP60/ClpP expression and/or binding is abnormal. Such conditions include, but are not limited to, cancer (e.g., prostate cancer and the like), cardiovascular diseases (e.g., coronary artery disease and the like), liver diseases (e.g., fatty liver disease and the like), and neurodegenerative diseases (e.g., Alzheimer's disease, dementia, and the like), and the like. An individual may an individual suspected of or having a condition where HSP60/ClpP expression and/or binding is abnormal.

An individual in need of treatment may have a tumor. The tumor may be from an androgen responsive, androgen non-responsive, and/or a neuroendocrine prostate cancer. A sample of the tumor may be obtained to determine levels of UPR^{mt} and/or HSP60 and/or ClpP. Examples of tumors that can be treated by the present methods or compositions comprising DCEM1 include, but are not limited to, breast adenocarcinoma, glioma, glioblastoma, medulloblastoma, multiple myeloma, melanoma, meningioma, ovarian carcinoma, prostate carcinoma, leukemia, lymphoma, colon carcinoma, pancreatic cancer, hepatic cancer, kidney cancer, sarcoma, and the like, and combinations thereof.

In various examples, the present disclosure discloses a method for treating an individual having or suspected of having cancer who has cells (e.g., has a tumor (e.g., tumor sample) having cells) expressing HSP60 and/or ClpP. A sample from the individual may be taken (e.g., tumor biopsy and/or blood sample) and HSP60 and/or ClpP levels may be measured. If the sample (e.g., tumor) is found to have elevated HSP60 and/or ClpP levels, a suitable, therapeutically effective amount of the composition comprising DCEM1 may be administered to the individual using an appropriate route (e.g., intratumoral, intravenous, intradermal injection, oral). The treatment may be carried out without first sampling HSP60 and/or ClpP levels. In various examples, the individual is administered one or more compounds (e.g., DCEM1) that disrupt the UPR^{mt} in cancer cells or a composition comprising one or more compounds that disrupt the UPR^{mt} in cancer cells.

The disclosure discloses a method for treating tumors, such as, for example, tumors that comprise cells expressing HSP60 and/or ClpP (e.g., prostate cancer cells). Such tumors may be referred to herein as "HSP60-expressing tumors", "ClpP-expressing tumors", and/or "HSP60/ClpP-expressing tumors". For example, the present disclosure discloses a method for reducing the size of a tumor and/or tumor cells; arresting the growth of a tumor and/or tumor cells; and/or reducing the rate of growth of a tumor and/or tumor cells (such as a tumor comprising HSP60/ClpP-expressing cells) or reducing any other symptom that is associated with an individual being afflicted with the tumor, all of which are considered as "treatment" comprising administering to an individual in need of treatment, a therapeutically effective amount of a composition comprising DCEM1, as described herein.

In an example, the current disclosure discloses a method of inhibiting growth and/or replication of prostate cancer cells comprising contacting the cells with the small molecule DCEM1 in a therapeutically effective amount and time sufficient to disrupt the UPR^{mt} by inhibiting the interaction of heat-shock protein 60 (HSP60) and caseinolytic protease proteolytic subunit (ClpP) in prostate cancer cells, wherein inhibiting HSP60-ClpP interaction results in inhibition of growth of the prostate cancer cells. In a further example, the prostate cancer cells to be treated can be of a hormone-sensitive, hormone-refractive, and/or neuroendocrine variant.

In an example, the method of the current disclosure discloses the treatment of an individual, comprising:
a) obtaining a sample from the individual (e.g., a tumor biopsy, blood);
b) determining the levels of UPR^{mt}, HSP60, and/or ClpP present in the sample and comparing the level of UPR^{mt}, HSP60, and/or ClpP to a reference standard. If the individuals UPR^{mt} and/or HSP60, and/or ClpP levels in the obtained sample is greater than the reference standard then;
c) administering to the individual a suitable amount of the small molecule DCEM1; and
d) monitoring the condition (e.g., cancer, such as, for example, tumor) in the individual following treatment.
In various examples, steps a, b, and d are optional.

One or more compositions described herein may be administered to an individual in need of treatment using any known method and route, including oral, parenteral, subcutaneous, intraperitoneal, intrapulmonary, intranasal, and intracranial injections. Parenteral infusions include intramuscular, intravenous, intraarterial, intraperitoneal, and subcutaneous administration. Topical and/or transdermal administrations are also encompassed.

In various examples, a subject in need of treatment is administered a therapeutically effective amount of a compound in a composition of the present disclosure. A dose of a therapeutically effective amount of a compound of the present disclosure may have a concentration of 10 nM to 10 mM (e.g., 100 µM), including all 0.1 nM values and ranges therebetween. In an example, a dose of a therapeutically effective amount of a compound in a composition of the present disclosure may have a concentration of 1-500 µM, 50-500 µM, 1-250 µM, 10-250 µM, 25-250 µM, 25-150 µM, 50-250 µM, or 50-150 µM.

In an example, an individual in need of treatment is administered a compound or a composition comprising the compound of the present disclosure as a single dose (e.g., a single administration step). Following a single dose, the individual's mitochondrial unfolded protein response activity is decreased for 1-120 hours (hr(s) or h) (e.g., 24-120 hours, 1-48 hours, 12-48 hours, or 24-48 hours), including all second values and ranges therebetween.

In an example, an individual in need of treatment is administered a compound or a composition comprising the compound of the present disclosure in multiple doses dose (e.g., multiple administration steps). Following the multiple doses, the individual's mitochondrial unfolded protein response activity is ameliorated for 1-120 hours (e.g., 24-120 hours, 1-48 hours, 12-48 hours, or 24-48 hours), including all second values and ranges therebetween.

A method may be carried out in a subject in need of treatment who has been diagnosed with or is suspected of having cancer (i.e., therapeutic use). A method can also be carried out in a subject who has a relapse or a high risk of relapse after being treated for cancer.

In an example, a method to inhibit the UPR^{mt} comprises: i) contacting a cancer cell (e.g., a cancer cell in a subject in need of treatment) using DCEM1, where the compound inhibits the interaction of HSP60 and ClpP.

In an example, a compound is used to inhibit the growth and/or replication of cancer cells (e.g., prostate cancer cells). Growth is inhibited by contacting the prostate cancer cells with a compound in an amount (e.g., 1 nM to 1 mM) and time sufficient to inhibit the UPR^{mt} in the cancer cells (e.g., prostate cancer cells), where the inhibition of UPR^{mt} results in inhibition of growth of the cancer cells (e.g., prostate cancer cells). The inhibition of growth may be better than the inhibition of growth caused by other compounds/treatments known in the art. Inhibition of growth refers to any decrease in growth/reproduction of a cell (e.g., the growth/reproduction of cancer cells).

In an example, a method of the present disclosure for treating cancer comprises: i) administering to a subject in need of treatment a composition of the present disclosure, where the compound binds to HSP60 and blocks HSP60 from interacting with ClpP and carrying out the mitochondrial unfolded protein response.

In an example, a method of the present disclosure for reducing the volume of a tumor comprises: i) administering to a subject in need of treatment a composition of the present disclosure, where the compound binds to HSP60 and blocks HSP60 from interacting with ClpP and carrying out the mitochondrial unfolded protein response.

In an example, a method of the present disclosure for preventing cancer metastasis comprises: i) administering to a subject in need of treatment a composition of the present disclosure, where the compound binds to HSP60 and blocks HSP60 from interacting with ClpP, and thus, blocking the mitochondrial unfolded protein response, which may prevent cancer metastasis.

A subject in need of treatment or individual in need of treatment may be a human or non-human mammal. Non-limiting examples of non-human mammals include cows, pigs, mice, rats, rabbits, cats, dogs, or other agricultural animals, pets, service animals, and the like. In various examples, the subject or individual is a male or has male reproductive organs. In various examples, the subject or individual has a prostate.

The steps of the method described in the various examples disclosed herein are sufficient to carry out the methods of the present disclosure. Thus, in an example, the method consists essentially of a combination of the steps of the methods disclosed herein. In another example, a method consists of such steps.

The following example is presented to illustrate the present disclosure. It is not intended to be limiting in any matter.

### EXAMPLE

The following is an example of use of DCEM1.

Upregulation of UPR^{mt} components associates with poor prognosis in PCa patients. PCa is a disease of aging prostate tissue that exhibits mitochondrial dysfunction, which activates a protective mechanism in mitochondria termed as the UPR^{mt}. To understand the potential impact of UPR^{mt} in PCa, publically available data sets were analyzed to characterize expression of key UPR^{mt} components and to test for correlations with patient outcomes. Transcript levels of HSP60 and ClpP were higher in prostate tumors compared to matched non-tumor prostate tissues **(****Figure 1A-B**). Higher expression of HSP60 and ClpP was observed in other types of tumor tissues compared to respective matched non-tumor tissues. It is interesting that transcript levels of HSP60 correlates with higher levels of its co-factor HSP10, and ClpP protease **(****Figure 1C-D****).** ClpP was highly expressed in PCa cell lines and is the most upregulated mitochondrial protease in PCa **(data not shown),** suggesting that HSP60 and ClpP expression may play an important role in PCa progression. cBioportal analysis also revealed that transcript levels of ClpP is the best correlated mitochondrial protease with HSP60 in prostate cancer. To validate at the protein level, HSP60 and ClpP protein expression in prostate tumors and normal tissues was determined by immunostaining. These data demonstrated significantly higher expression of HSP60 and ClpP in prostate tumors compared to matched normal tissues **(****Figure 1E****).** Transcript and protein expression of HSP60 and its co-factor HSP10 along with ClpP were highly upregulated among all tested PCa cell lines compared to non-malignant prostate epithelial RWPE-1 and HPC161T cells **(data not shown).** Similar data were observed in transgenic adenocarcinoma of mouse prostate tumors **(data not shown).** To understand the impact of UPR^{mt} in PCa, the TCGA PRAD dataset was analyzed to correlate PCa patient survival with expression of key components of UPR^{mt}. We observed that increased expression of HSP60 and its cofactor HSP10 associates with poor overall survival of PCa patients **(****Figure 1F****).** These data demonstrate that key components of UPR^{mt} are upregulated and associate with PCa progression and poor prognosis.

HSP60 and ClpP silencing inhibit PCa cell survival and proliferation. Activation of UPR^{mt} plays an important role in restoring mitochondrial function and cancer cell survival. Clonogenic assay demonstrated that HSP60 or ClpP-silencing reduced the clonogenicity of several PCa cell lines **(****Figure 2A****).** Genetic silencing of HSP60 and ClpP in PCa cells downregulated the expression of c-Myc as well as EZH2, the two major drivers of PCa growth, proliferation, and poor prognosis **(****Figure 2B-D****).** Because HSP60 and ClpP are required for maintenance of mitochondrial function and ATP is an energy source for proliferating cancer cells, the oxygen consumption rate (OCR) in HSP60/CLpP silenced cells was analyzed. Silencing of either HSP60 or ClpP inhibited basal and maximal respiratory rate of PCa cells, suggesting that both proteins are crucial to maintain mitochondrial function **(****Figure 2E****).** ATP levels were also significantly suppressed in HSP60 and ClpP-silenced PCa cells **(****Figure 2F****)** along with the downregulation of OXPHOS subunits **(data not shown).** Using CRISPR Cas9, one allele of HSD1 gene was deleted in DU145 PCa (DU145 HSP60^{+/-}) cells because HSPD1 knockout is embryonic lethal. Similar to HSP60-silencing, reducing HSP60 protein by deleting one copy of HSPD1 reduced various OXPHOS proteins and ATP levels **(data not shown).** Silencing of HSP60 or ClpP increased mitochondrial ROS production, but not mitochondrial mass **(data not shown).** These data demonstrate silencing of UPR^{mt} components compromises mitochondrial function as expected. Loss of mitochondrial function may account for decreased clonogenicity of HSP60 or CLpP silenced PCa cells.

Silencing/knockout of key UPR^{mt} components reduces tumor burden *in vivo.* To determine the physiological relevance of HSP60 and ClpP on tumor growth, these two genes were silenced in highly invasive androgen-independent PCa cell line PC-3 and performed subcutaneous xenograft studies. Significant reduction in tumor growth was observed upon silencing of HSP60 or ClpP, HSP60-silencing showed higher efficacy in reducing tumor burden **(****Figure 3A-C****).** This may be due to the observation that HSP60 silencing also causes a reduction in ClpP, but ClpP silencing does not affect HSP60 levels. Ki67 staining, a marker for cell proliferation, was reduced in HSP60-silenced and ClpP-silenced tumors **(****Figure 3D****),** thus showing reduced tumor cell proliferation. HSP60-silencing caused mitochondrial OXPHOS dysfunction as expression of nuclear encoded proteins required for complexes I, II, III, IV, and V were reduced in HSP60-silenced PC-3 cell xenograft samples **(data not shown).** Defective OXPHOS may have compromised mitochondrial structure and function causing increased cell death in the tumors.

To test the importance of HSP60 in more physiological PCa models, floxed *HSP60* alleles were bred into a genetically engineered mouse model of highly aggressive neuroendocrine PCa in which the *Pten, Rb1,* and *Trp53* tumor suppressor genes were deleted specifically in prostate epithelial cells (TKO). TKO animals have a median survival of 16 weeks. All animals at this age were euthanized as they are expected to have readily detectable prostate tumors. Deletion of both HSP60 alleles significantly decreased prostate tumor weight and volume in TKO animals **(****Figures 3E-G****).** Deletion of only one HSP60 was sufficient to significantly reduce tumor burden in TKO animals. Deletion of both HSP60 alleles alone does not yield a detectable prostate phenotype distinguishable from wild type mice. Thus malignant prostate tissue requires higher levels of HSP60 to maintain growth and survival compared to non-malignant tissue. It was confirmed decreased expression of HSP60 or HSP60 knockout in TKO HSP60 F/+ and TKO HSP60 F/F prostate tissue **(****Figure 3H****).** As noted with PC-3 cell xenografts, HSP60 knockout in TKO prostatic tissue downregulated ClpP expression **(****Figure 3H****).** TKO tumors expressed synaptophysin (SYP) along with low expression of epithelial marker cytokeratin 8/18 (CK 8/18), indicating neuroendocrine (NE) phenotype. Both 50% and complete HSP60 knockout in TKO downregulated SYP and upregulated CK 8/18 expression, suggesting that reduction or HSP60 KO inhibited the PCa NE transformation occurring in these mice **(****Figure 31****).** Downstream targets c-Myc and EZH2 expression were also reduced or abolished **(****Figure 3J****)** upon ablation of HSP60. Energy demands were diminished due to decreased tumor growth evidenced by decreased ATP levels were reduced upon HSP60 abrogation in TKO prostatic tissue **(****Figure 3K****),** consistent with increased mitochondrial dysfunction. Together, these observations indicate HSP60 deficiency and consequent mitochondrial dysfunction decreased PCa progression *in vivo.*

HSP60 regulates expression of ClpP via c-Myc but not vice versa. Decreased ClpP expression was observed in HSP60 KO TKO prostatic tissue **(****Figure 3H****),** which is consistenet with recent observation that HSP60 KO abolishes ClpP expression in mouse heart tissue. To understand the interplay between HSP60 and ClpP, HSP60 or ClpP was silenced in PCa cells. HSP60-silencing greatly reduced ClpP expression, ClpP oligomerization, and ClpP activity in PCa cells **(****Figure 4A-D****).** HSP60-silencing did not affect the expression of LONP1, another mitochondrial matrix located ATP-dependent AAA⁺ protease **(data not shown).** Intriguingly, the level of HSP60 and its oligomerization was not modulated by ClpP silencing **(****Figure 4A-C****).** To establish that HSP60 drives the activation of UPR^{mt} leading to increased expression of ClpP, HSP60 was overexpressed in PCa cells and observed elevated expression of ClpP **(****Figure 4E****).** However, ClpP overexpression did not modulate HSP60 exprerssion in PCa cells **(****Figure 4F****).** Because HSP60 knockdown depletes ClpP at protein and mRNA levels **(data not shown),** ENCODE database was searched, and it was observed that c-Myc as a transcription factor regulates ClpP expression. To confirm that c-Myc is a regulator for ClpP expression, PCa cells were treated with c-Myc pharmacological inhibitor 10058-F4 and analyzed ClpP expression levels. These results demonstrated that c-Myc inhibitor downregulate ClpP expression without affecting HSP60 expression **(****Figure 4G****).** To confirm c-Myc regulates ClpP expression, ChIP primers were designed using ENCODE database, and performed ChIP assay with c-Myc antibody in HSP60 knockdown PCa cells. Reduced c-Myc binding to ClpP promoter was observed in HSP60 knockdown LNCaP and PC-3 cells **(****Figure 4H****).** Knocking down HSP60 in PCa cells downregulated c-Myc expression **(****Figure 4I****).** c-Myc (primarily a transcription factor) was observed in the mitochondrial compartment of PCa cells, suggesting that HSP60-ClpP chaperonin system is crucial for maintenance of the c-Myc protein **(****Figure 4J****).** Together, these data suggest that HSP60 regulates ClpP expression via downregulating c-Myc.

UPR^{mt} components HSP60 and ClpP localize and interact in mitochondria. Mitochondrial protein folding machinery requires oligomerization of HSP60 and its interaction with HSP10 to maintain mitochondrial proteostasis. In contrast, unfolded proteins are degraded by ClpP proteases to attenuate stress and maintain protein homeostasis under that stress. To understand the functional significance of these mitochondrial proteins, the localization of HSP60 and ClpP in various PCa cells was evaluated. Immunofluorescence and biochemical analysis clearly demonstrated that HSP60 and ClpP co-localize in the mitochondrial matrix **(data not shown).** Co-immunoprecipitation analysis demonstrated that HSP60 interacts with ClpP in multiple PCa cell lines **(****Figure 5A****).** Proximity ligation assay (PLA) using PCa cells and prostate-specific tissue micro arrays (TMAs) also demonstrated *in vivo* interaction between HSP60 and ClpP in PCa cells and primary prostate tumors **(****Figure 5B and 5C****).** HSP60 and HSP10 PLA functions as a positive control for HSP60-interacting proteins **(data not shown).** HSP60 did not interact with LONP1, another mitochondrial proteases in PCa cells **(data not shown).** As expected, control IgGs did not show PLA signals **(data not** shown). Knocking down HSP60 abrogated or diminished PLA signal confirming the specificity of PLA **(data not shown).**

To study the dynamics of HSP60-ClpP interaction, several HSP60 mutants were created and co-transfected with ClpP expressing plasmid in PCa cells. As shown in **Figure 5D****,** deletion of mitochondrial localization signal (HSP60^{N-Del}) and/or apical domain (HSP60^{ΔApi}) from HSP60 protein abolished its interaction with ClpP. HSP60 oligomerizes to tetradecamer structure to exert its biological function and ClpP proteins also exist in oligomeric state. It was next explored whether this interaction is due to protein-protein interaction between HSP60 and ClpP or the association between these two protein complexes is required. A HSP60^{D3G} mutant that disrupts HSP60 oligomerization was generated. It was observed that the HSP60^{D3G} mutant reduced FRET efficiency, reflecting reduction in HSP60-HSP60^{D3G} interaction compared to HSP60-HSP60 interaction, suggesting that the HSP60^{D3G} mutant shows reduced HSP60 oligomerization **(****Figure 5E****).** Interestingly, ClpP interaction with HSP60 was significantly reduced upon D3G mutation in HSP60 **(****Figure 5F and G****).** These findings suggest that HSP60 oligomerization is required for its interaction with ClpP and may influence the level and function of ClpP. In addition, co-immunoprecipitation also confirms HSP60-ClpP interaction in TKO prostatic tumor tissue **(****Figure 5H****).** Together, these findings provide strong evidence that HSP60 co-localize and interacts in its oligomeric form with ClpP in mitochondrial matrix through its apical domain, which may provide novel insight on interplay between UPR^{mt} components in PCa.

Identification of novel inhibitor of HSP60-ClpP interaction that disrupts UPR^{mt} function and induces PCa cell death. Having shown that two arms of UPR^{mt}, HSP60 chaperonin and ClpP protease interact and play critical role in PCa cell survival, an *in silico* analysis was performed and a small molecule library (developed by Enamine Ltd) was screened to identify drugs targeting the apical domain of HSP60 critical for its interaction with ClpP. 9 compounds (A-I) were identified and screened for their cell death inducing potential in PCa cells **(data not shown).** It was observed that compound A (referred to as DCEM1) caused robust cell death in PCa cells with no effect on non-carcinoma prostate epithelial RWPE-1 cells **(****Figure 9****).** The molecular docking of DCEM1 into HSP60 protein is shown in **Figure 6A****.** Co-immunoprecipitation demonstrated that DCEM1 inhibited HSP60-ClpP interactions in a dose dependent manner **(****Figure 6B****).** PLA also showed that DCEM1 efficiently inhibited HSP60-ClpP interaction in PCa cells **(data not shown).** To understand physiological consequence of the inhibiting HSP60-ClpP interaction, mitoROS and poly-Ub protein levels were analyzed. mitoROS upregulation accompanied by increased accumulation of poly-Ub proteins was observed in response to DCEM1 treatment **(****Figure 6C and D****),** suggesting that inhibition of HSP60-ClpP interaction interferes with mitochondrial protein homeostasis and affects PCa cell survival. Indeed, robust caspase activity and apoptotic cell death was observed **(****Figure 6E and F****).** Colony forming ability of PCa cells was inhibited by DCEM1 treatment **(data not shown).** DCEM1 treatment induced mitoROS and mitoMass in TKO PCa cells (established from TKO mouse prostate tumor tissue) as well as induced robust apoptotic cell death **(data not shown).** MitoROS quencher SKQ1 inhibited DCEM1-induced cell death, suggesting that DCEM1-induced cell death is caused by mitoROS production **(****Figure 6G****).** NAC pre-treatment antagonizes DCEM1-induced cell death in PCa cells confirming the pro-oxidant properties of DCEM1 **(data not shown).** Severe mtDNA damage upon DCEM1 exposure to PCa cells further provided evidence that disruption of HSP60-ClpP interaction contributes to mitoROS production and mitochondrial dysfunction **(****Figure 6H****).** DCEM1 treatement failed to alter the expression of UPR^{mt} components including HSP60, HSP10, ClpP and LONP1 **(****Figure 6I****).** These findings suggest that disruption HSP60-ClpP interaction derailed the prosurvival function UPR^{mt} in PCa cells.

Since HSP60 chaperonin system is crucial for maintaining mitochondrial homeostasis in normal cells, a novel method, **Mitochondrial Chaperonin Activity Assay** (MiCAA), was developed to analyze chaperonin activity in live cells using flow cytometry to evaluate whether DCEM1 modulates mitochondrial chaperonin activity. MiCAA demonstrated that DCEM1 did not modulate mitochondrial chaperonin activity in PCa cells **(data not shown),** whereas ETB and mizoribine, known chaperonin activity inhibitors, significantly inhibited in PCa cells **(****Figure 10B****).** Genetic approaches using HSP60-specific siRNAs demonstrate that knocking down HSP60 significantly inhibited mitochondrial chaperonin activity in PCa cells **(****Figure 10C****).** Importantly, DCEM1 did not alter chaperonin function of HSP60 in mammalian cells highlighting its potential therapeutic implication in PCa.

Abrogation of HSP60-ClpP interaction by DCEM1 induces metabolic stress in PCa cells. To dissect the underlying mechanism of mitoROS production upon disruption of HSP60-ClpP interaction, OXPHOS Complex I was inhibited using rotenone or OXPHOS Complex III by antimycin-A, the known sources of mitoROS production. It was observed that DCEM1-induced mitoROS production was not attenuated by inhibition of Complex I or III **(****Figure 7A****).** Overexpression of ClpP inhibited DCEM1-induced mitoROS production and DEVDase activity in PCa cells, suggesting that accumulation of unfolded proteins contributes mitoROS **(****Figure 7B-D****).** Given the importance of UPR^{mt} in maintaining mitochondrial protein homeostasis and functions, it was hypothesized that disruption of HSP60-ClpP interaction promotes metabolic stress in PCa cells. Indeed, it was observed that DCEM1 treatment robustly depolarized mitochondrial membrane along with reduction of cellular ATP **(****Figure 7E** **and** **F****).** It was observed that DCEM1 abrogates retrograde signaling as evidenced by downregulation of nuclear encoded subunits of OXPHOS Complexes accompanied by reduction in oxygen consumption rate (OCR) in PCa cells **(****Figure 7G and H****).** Since ATP depletion/reduction induces autophagy, autophagy activation in PCa cells was analyzed in response to DCEM1 treatment. Robust increase in p-AMPKα levels and LC3B cleavage were observed in PCa cells upon DCEM1 treatment. The activation of AMPK pathway was further confirmed by enhanced phosphorylation at serine 555 residue and decreased phosphorylation at serine 757 residue of ULK1. DCEM1 treatment also inhibited mTOR signaling in PCa cells **(****Figure 7I****).** Taken together, these results suggest that pharmacological inhibition of HSP60-ClpP interaction induces autophagy in PCa cells by activating AMPK and inhibiting mTOR pathways.

Ablation of HSP60-ClpP interaction by DCEM1 downregulates c-Myc, EZH2 and AR as well as inhibits tumor growth *in vivo.* It was observed that DCEM1 downregulates c-Myc, EZH2, and AR signaling in PCa cells. Decrease in PSA levels also established that DCEM1 disrupt the major drivers of PCa growth and progression **(****Figure 8A and B****).** The effect of DCEM1 on 22RV1 (AR positive) and PC-3 (AR negative) xenografts growth in SCID mice was analyzed. It was observed that 60 mg/kg bw dose effectively inhibited tumor volume and weight in 22RV1 **(****Figure 8C and D****)** and PC-3 xenografts **(****Figure 8G and H****).** DCEM1 treatment induced caspase-3 (DEVDase) activity in both 22RV1 and PC-3 xenograft tumors **(****Figure 8E** **and** **I****).** DCEM1 treatment downregulated expression of Ki67, c-Myc, EZH2 and AR in 22RV1 xenograft confirming the *in vivo* efficacy of DCEM1 **(****Figure 8F****).** DCEM1 treatment inhibited HSP60-ClpP interaction as suggested by PLA confirming the availability and efficacy of DCEM1 in PC-3 xenograft tumors **(****Figure 8J****).** To evaluate the efficacy of DCEM1 on spontaneous prostate tumor growth and incidence in a pre-clinical PCa model, PTEN:Rb:p53 knockout (TKO) animals were treated with DCEM1 twice weekly from 10 weeks to 16 weeks of age. As shown in **Figure 8K****,** DCEM1 treatment significantly inhibited prostate tumor growth in TKO mice as evidenced by decreased GU weight and tumor volume using MRI images **(****Figure 8L and M****).** DCEM1 treatment downregulated HSP60 and ClpP expression in TKO prostatic tissue along with depletion of c-Myc and EZH2 confirming the notion that efficacy of DCEM1 is facilitated by depletion of proto-oncogenes **(****Figure 8Nand O****).** DCEM1 treatment downregulated SYP and up regulated CK 8/18 expression in TKO tumor tissues, suggesting that DCEM1 prevent the acquisition of neuroendocrine phenotype in TKO tumors **(****Figure 8P****).** Overall, these results suggest that HSP60 and ClpP interaction is essential for maintenance of protein homeostasis. Inhibition of HSP60-ClpP interaction hyperactivates UPR^{mt} in PCa cells, which can be an attractive therapeutic strategy for PCa treatment regardless of AR status.

Discussion. Development of resistance to AR targeted therapy in advanced PCa associates with tumor heterogeneity, which may arise due to varying levels of AR or occurrence of AR variants in prostate tumor cells or emergence of NEPC. THe HSP60-ClpP axis was identified as a new target for developing novel PCa therapeutics. The HSP60-ClpP axis is highly upregulated in the majority of prostate tumor tissues, suggesting that this axis provides a novel target for AR-sufficient and AR-deficient/defective prostate tumors. Cancer cells may undergo mitohormesis by constitutively activating cytoprotective mechanisms like UPR^{mt}. It was hypothesized that perturbation of UPR^{mt} would have deleterious effect on growth and progression of PCa. Based on the findings that HSP60 reduces expression of ClpP along with recent findings, inhibiting HSP60 function alone may be sufficient for tumor regression and prevention of recurrence in PCa.

HSP60 and HSP10 are integral components of protein folding machinery in mitochondria, whereas ClpP protease plays a pivotal role in degradation of unfolded proteins. Although other proteases actively coordinates with ClpP proteases in maintaining cellular and mitochondrial proteostasis, only ClpP expression and function were modulated by HSP60 silencing or inhibition. These findings provide evidence that ClpP protease is the key HSP60 client protein from proteolysis machinery to regulate UPR^{mt} in PCa. Drastic decrease in ClpP level and reduced cellular viability upon HSP60-silencing suggests that HSP60 renders its prosurvival function at least in part via ClpP. Increased coexpression of HSP60 and ClpP in PCa cells as well as in clinical PCa specimens suggests that HSP60-ClpP axis is a novel viable target for PCa therapy. The *en silico* analysis identified several domains of HSP60 and this study showed that HSP60 interacts with ClpP via its apical domain. Deletion of mitochondrial localization signal or inhibition of oligomerization of HSP60 prevent its interaction with ClpP, suggesting that HSP60 interact with ClpP in its oligomeric form via its apical domain in mitochondria.

Although HSP60-silencing renders higher anticancer activities compared to ClpP-silencing, thus providing an opportunity to disrupt the chaperoning activity of HSP60 or HSP60-ClpP interaction for developing potent anticancer agents. HSP60 and ClpP are required for normal and cancer cell survival/function, thus HSP60 knockout or HSP60-silencing or ClpP-silencing is not feasible option in clinics. HSP60 chaperonin activity is also required for normal cellular functioning. Thus, inhibiting HSP60 chaperonin function may have serious deleterious effect that was observed with phase II clinical trials of HSP90 inhibitor on metastatic PCa. This suggest that disrupting HSP60-ClpP interaction may represent a novel and safe target for PCa therapy. Identification of DCEM1 that blocks HSP60-ClpP interaction enhances PCa cell death and inhibit tumor growth *in vivo* in multiple xenografts and in vivo PCa models represent an unique small molecule for PCa therapy.

A reason for ClpP downregulation is that HSP60-silencing may destabilize ClpP leading to its degradation. Reduced ClpP mRNA upon HSP60-silencing suggests that degradation of ClpP protease is not the sole reason for reduction of ClpP protein. Downregulation of c-Myc by genetic or pharmacological ablation of HSP60 and identification of c-Myc as a transcriptional regulator of ClpP may indicate the involvement of mitochondria-to-nucleus (retrograde) signaling. Thus, it is envisioned that inhibition of HSP60-ClpP interaction by DCEM1 will disrupt mitochondrial homeostasis leading to inhibition of PCa progression and prevention of PCa recurrence.

The majority of mitochondrial proteins are encoded by nuclear DNA and newly synthesized polypeptides are translocated to mitochondria for proper folding. Cancer cells require increased protein synthesis to meet the demands of increased cellular proliferation and accompanying mitochondrial stress, causing activation of UPR^{mt} leading to increased synthesis of HSP60 and ClpP. Increased levels of these two key components of the UPR^{mt} may ultimately influence mitochondrial OXPHOS function leading to increased ROS production, which further enhance UPR^{mt}. Thus there is a continued demand for UPR^{mt} activation in attenuating persistent mitochondrial stresses during PCa tumorigenesis. These findings establish that pharmacological and genetic inhibition of HSP60 and ClpP function decreased the key tumor promoting proteins such as c-Myc and EZH2, ultimately leading to inhibition of cellular proliferation and enhancement of cancer cell death. On the other hand, blocking HSP60-ClpP interaction by DCEM1 will induce metabolic stress causing burst of mitochondrial ROS, shifting balance towards pro-oxidants based therapeutic strategy. Activation of AMPK, inhibition of mTOR pathways, and burst of oxidative stress in response to DCEM1 suggest that abrogation of HSP60-ClpP interaction is a driving force for OXPHOS defects and metabolic stress in PCa.

Although cancer cells prefer aerobic glycolysis for energy, continual production of ATP via OXPHOS system is still required for rapid cancer cell survival and growth. ClpP along with LonP1 degrades Complex I during stress to alleviate mtROS, thus promoting cell survival. HSP60-silencing or inhibition of HSP60-ClpP interaction by DCEM1 contribute to mtROS buildup and mitotoxicity due to inhibition of ClpP function or ClpP-deficiency because ClpP is no longer available/functional in alleviating mtROS production and degrading unfolded protein in mitochondria leading to collapse of mitochondrial function and homeostasis. How HSP60 regulates expression of nuclear-encoded OXPHOS subunits and other mitochondrial machinery is not clearly understood, but this study indicates that HSP60 is a key regulator of mitochondrial-nuclear crosstalk. Mitochondrial proteases such as ClpP trigger mitochondria-to-nuclear signaling pathway and UPR^{mt} activation. Inhibition of ClpP expression and function by HSP60-silencing or HSP60 inhibitor such as DCEM1 may abolish mito-nuclear crosstalk leading to inhibition of retrograde signaling. Inhibition of HSP60 renders mitochondria in a fragile and dysfunctional state leading to enhanced apoptosis and blockage of cellular proliferation. Therefore, chemotherapeutic agents along with HSP60 inhibition may provide a novel combination for therapeutic benefits in PCa. Indeed, it was observed that docetaxel is more efficacious in inducing cell death in HSP60-silenced cells compared to mock cells. Therefore, OXPHOS collapse upon HSP60 and ClpP-silencing or by DCEM1 treatment enhances robust cell death and may be a major reason why cell proliferation and viability is reduced.

Altogether, it was determined that PCa cells display oncogene addiction towards UPR^{mt} components HSP60 and ClpP, as silencing of either proteins inhibit PCa growth and progresison. It was also showed that HSP60 and ClpP are interacting proteins and disrupting their interaction with each other had the same effect as silencing. This observation may be of utmost importance as chaperonin activity of HSP60 and protease activity of ClpP are needed by normal cells as well to maintain mitochondrial protein homeostasis but the PLA data described herein showed this interaction is not significant in normal prostate tissue. Therefore, targeting HSP60-ClpP axis in PCa (which is prevalent in PCa regardless of AR status) is a viable option and is expected to hinder PCa progression.

## Claims

1. A composition for use in a method for treating an individual in need of treatment having a condition in which HSP60/ClpP expression and/or binding is abnormal, the use comprising administering to the individual in need of treatment a therapeutically effective amount of the composition comprising a compound having the following structure: 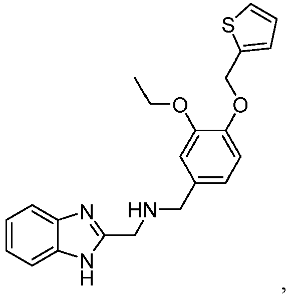 wherein the condition is a cancer chosen from prostate cancer, leukemia, lung cancer, dermatological cancer, premalignant lesions of the upper digestive tract, malignancies of the brain, malignancies of the breast, and combinations thereof.

2. The composition for use of claim 1, wherein the cancer is prostate cancer

3. The composition for use of claim 2, wherein the prostate cancer is "castration-resistant" prostate cancer.

4. The composition for use of claim 2, wherein the prostate cancer is androgen receptor-responsive prostate cancer or androgen receptor-nonresponsive prostate cancer.

5. The composition for use of claim 2, wherein the prostate cancer is a neuroendocrine prostate cancer.

6. The composition for use of claim 1, wherein the method is used in combination with surgery, radiation therapy, chemotherapy, photodynamic therapy, and/or immunotherapy.

7. The composition for use of claim 6, wherein the chemotherapy comprises administering docetaxel.

## Patentansprüche

1. Zusammensetzung für die Verwendung in einem Verfahren zur Behandlung eines behandlungsbedürftigen Individuums, das ein Leiden hat, in dem HSP60/ClpP- Expression und/oder -Bindung abnormal sind, wobei die Verwendung das Verabreichen an das behandlungsbedürftige Individuum einer therapeutisch wirksamen Menge der Zusammensetzung umfasst, die eine Verbindung umfasst, die die folgende Struktur aufweist: 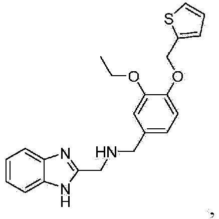 wobei das Leiden ein Krebs ist, der aus Folgenden ausgewählt ist: Prostatakrebs, Leukämie, Lungenkrebs, Hautkrebs, prämalignen Läsionen des oberen Verdauungstrakts, Malignomen des Gehirns, Malignomen der Brust und Kombinationen davon.

2. Zusammensetzung für die Verwendung nach Anspruch 1, wobei der Krebs Prostatakrebs ist.

3. Zusammensetzung für die Verwendung nach Anspruch 2, wobei der Prostatakrebs "kastrationsresistenter" Prostatakrebs ist.

4. Zusammensetzung für die Verwendung nach Anspruch 2, wobei der Prostatakrebs Androgenrezeptor-responsiver Prostatakrebs oder Androgenrezeptor-nicht-responsiver Prostatakrebs ist.

5. Zusammensetzung für die Verwendung nach Anspruch 2, wobei der Prostatakrebs ein neuroendokriner Prostatakrebs ist.

6. Zusammensetzung für die Verwendung nach Anspruch 1, wobei das Verfahren in Kombination mit Operation, Strahlentherapie, Chemotherapie, photodynamischer Therapie und/oder Immuntherapie verwendet wird.

7. Zusammensetzung für die Verwendung nach Anspruch 6, wobei die Chemotherapie das Verabreichen von Docetaxel umfasst.

## Revendications

1. Composition destinée à une utilisation dans une méthode de traitement d'un individu nécessitant un traitement et présentant une condition dans laquelle l'expression et/ou la liaison de HSP60/ClpP est anormale, l'utilisation comprenant l'administration à l'individu nécessitant un traitement d'une quantité thérapeutiquement efficace de la composition comprenant un composé ayant la structure suivante : 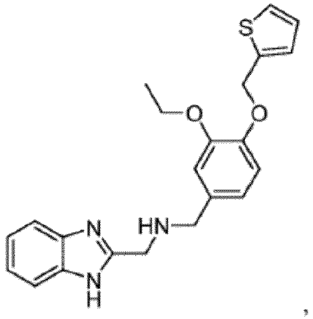 où la condition est un cancer choisi parmi le cancer de la prostate, la leucémie, le cancer du poumon, un cancer dermatologique, les lésions pré-malignes du tractus digestif supérieur, les tumeurs malignes du cerveau, les tumeurs malignes du sein et des combinaisons de ceux-ci.

2. Composition destinée à une utilisation selon la revendication 1, le cancer étant le cancer de la prostate.

3. Composition destinée à une utilisation selon la revendication 2, le cancer de la prostate étant un cancer de la prostate « résistant à la castration ».

4. Composition destinée à une utilisation selon la revendication 2, le cancer de la prostate étant un cancer de la prostate sensible aux récepteurs aux androgènes ou un cancer de la prostate non sensible aux récepteurs aux androgènes.

5. Composition destinée à une utilisation selon la revendication 2, le cancer de la prostate étant un cancer neuroendocrine de la prostate.

6. Composition destinée à une utilisation selon la revendication 1, la méthode étant utilisée en association avec une chirurgie, une radiothérapie, une chimiothérapie, une thérapie photodynamique et/ou une immunothérapie.

7. Composition destinée à une utilisation selon la revendication 6, la chimiothérapie comprenant l'administration de docétaxel.
